Europäisches Patentamt

European Patent Office

Office européen des brevets

(19)

(11) EP 0 524 173 B1

(12) EUROPEAN PATENT SPECIFICATION

(45) Date of publication and mention
of the grant of the patent:
**19.06.1996 Bulletin 1996/25**

(21) Application number: **91900632.0**

(22) Date of filing: **23.10.1990**

(51) Int Cl.6: **C12N 15/12**, **A61K 39/395**,
**A61K 35/12**, **C07K 14/705**,
**G01N 33/68**, **C12Q 1/68**

(86) International application number:
**PCT/US90/06124**

(87) International publication number:
**WO 91/06646 (16.05.1991 Gazette 1991/11)**

(54) **SOLUBILIZATION AND PURIFICATION OF THE GASTRIN RELEASING PEPTIDE RECEPTOR**

SOLUBILISIERUNG UND REINIGUNG DES REZEPTORS FÜR DAS GASTRIN-FREISETZENDE PEPTID

SOLUBILISATION ET PURIFICATION DU RECEPTEUR DE PEPTIDES DE LIBERATION DE GASTRINE

(84) Designated Contracting States:
**AT BE CH DE DK ES FR GB GR IT LI LU NL SE**

(30) Priority: **24.10.1989 US 426150**
**05.06.1990 US 533659**

(43) Date of publication of application:
**27.01.1993 Bulletin 1993/04**

(73) Proprietor: **BERLEX LABORATORIES, INC.**
**Cedar Knolls, NJ 07927-2094 (US)**

(72) Inventors:
- **FELDMAN, Richard, I.**
**El Cerrito, CA 94530 (US)**
- **WU, James, M.**
**El Cerrito, CA 94530 (US)**
- **MANN, Elaina**
**San Leandro, CA 94577 (US)**
- **LAROCCA, Anne**
**San Leandro, CA 94577 (US)**
- **JENSON, James, C.**
**Moraga, CA 94556 (US)**

(74) Representative: **Thomson, Paul Anthony**
**Potts, Kerr & Co.**
**15, Hamilton Square**
**Birkenhead Merseyside L41 6BR (GB)**

(56) References cited:
- **BIOCHEMISTRY, vol. 29, 1990, American Chemical Society, US; L. NALDINI et al., pp. 5153-5160/**
- **JOURNAL OF BIOLOGICAL CHEMISTRY, vol. 263, no. 1, 05 January 1988, American Society for Biochemistry & Molecular Biology Inc., US; J. MAZELLA et al., pp. 144-149/**
- **JOURNAL OF BIOLOGICAL CHEMISTRY, vol. 262, no. 23, 15 August 1987, American Society for Biochemistry & Molecular Biology Inc., US; R.M. KRIS et al., pp.11215-11220/**
- **JOURNAL OF BIOLOGICAL CHEMISTRY, vol. 265, no. 28, 05 October 1990, American Society for Biochemistry & Molecular Biology Inc., US; R.I. FELDMAN et al., pp. 17364-17372/**
- **Ann NY Acad Sci 511 (1987) 256**
- **"Brain Receptor Methodologies, Part A", 1984, Acad Press, Inc., Marangos et al (eds.), pp. 75-93**
- **"A Guide to the Properties and Uses of Detergents in Biology and Biochemistry", 1988, Neugebauer, Calbiochem Co, pp. 1-3**
- **Methods Enzymology 104 (1984) 329-339**
- **Proc Natl Acad Sci USA 77 (1980) 6368**

## Description

Growth factors are involved in numerous physiological and pathological processes. An increasing number of small regulatory peptides have been discovered in the neural and neuroendocrine cells of mammalian tissues. More recent evidence has pointed to the role of neuropeptides in the regulation of animal cell growth, and in particular to the action of mitogenic peptides in the Swiss 3T3 cell system. One of the first neuropeptides studied was the tetradecapeptide bombesin which was originally isolated from amphibian skin, Anastasi et al., Experientia 27:166-167 (1971). Bombesin is structurally related to several endogenous mammalian peptides, the first to be characterized being gastrin releasing peptide.

Gastrin releasing peptide (GRP) is a 27 amino acid peptide having the following sequence in humans: Val-Pro-Leu-Pro-Ala-Gly-Gly-Gly-Thr-Val-Leu-Thr-Lys-Met-Tyr-Pro-Arg-Gly-Asn-His-Trp-Ala-Val-Gly-His-Leu-Met-NH2. GRP is of significant interest because of its presumed ability to function as an autocrine growth factor in the pathogenesis of cancer. In particular, GRP has been found to promote growth of human small cell lung carcinoma (SCLC). GRP binding to cell surface receptors is thought to stimulate cellular growth by promoting the hydrolysis of phosphatidyl inositides and by activation of protein kinase C. A large number of biological responses to GRP have been observed including stimulation of Na+/H+ antiport, mobilization of intracellular Ca2+, transient expression of c-fos and c-myc proto-oncogenes, induction of tyrosine kinase activity, elevation of DNA synthesis and promotion of cell division.

The role of GRP in maintaining the growth of SCLC suggests that effective therapeutic agents could be developed that interrupt the autocrine growth cycle by inactivating GRP or inhibiting its receptor. The active site of GRP is the C-terminal region which binds high affinity receptors on SCLC membranes. Blocking this binding can inhibit SCLC growth. This has already been accomplished with monoclonal antibodies to bombesin which bind to the active site on GRP, thus inactivating the peptide, Cuttitta et al., Nature 316:823- 826 (1985).

Another means to block GRP binding to its receptor, and therefore to treat SCLC, is to inhibit the receptor itself. This can be accomplished by use of agents which bind to the GRP receptor and act as antagonists. Antagonists can normally be found once the receptor has been pharmacologically defined, as is the case with the GRP receptor. Testing of potential receptor antagonists has been made much easier with the development of highly automated assay methods. Unfortunately, these systems require purified GRP receptor in an active form, which has not been readily attainable. This problem can be overcome by use of the recombinant receptor. Along with providing an improved renewable source of the receptor from a specific source, using the recombinant GRP receptor in screening for GRP receptor reactive drugs also has the following advantages: potentially greater number of receptors per cell giving greater yield of reagent and higher signal to noise ratio in assays; and receptor subtype specificity (theoretically giving greater biological and disease specificity).

Cross-linking of the GRP receptor to bound radio-labeled GRP has been used to visualize the GRP receptor-ligand conjugate on SDS-PAGE and to deduce certain other characteristics of the receptor Rosengurt et al., PCT/GB88/00255. However, the technique used did not involve isolation of the receptor but rather involved characterization of a modified form of the receptor protein. Unfortunately, in order to characterize the structural properties of the GRP receptor in greater detail and to understand the mechanism of action at the molecular level, the receptor needs to be purified. For some applications, it is essential to purify the receptor in an active state which maintains the binding activity of the receptor. These include the generation of antibodies against active receptor epitopes, structural studies of the ligand binding site, and the use of the purified receptor for screens for agonists and antagonists of GRP binding.

J. Biol. Chem. 263 (1988) 144 - 149 discloses methods for the solubilisation and characterisation of active neurotensin receptors from mouse brain using 3-[(3-cholamidopropyl)dimethylammonio]-1-propane sulphonate or sulphonic acid and cholesterol hemisuccinate.

J. Biol. Chem. 262 (1987) 11215 - 11220; abstract, discloses the bombesin or gastrin-releasing peptide (GRP) receptor as well as disclosing that the receptor is a glycoprotein found on the surface of murine and human cells.

To date, few receptors have been isolated and characterized in their active form. There are two main reasons for this. First, the amount of receptor present in most tissues is minute and second, the receptor must often be solubilized from membranes with detergents that can perturb the structure of the protein. Further compounding these difficulties is the unpredictable nature of receptors in that the method for successfully solubilizing one protein receptor may not be successful for a different protein receptor.

This invention pertains to the solubilization of the active GRP receptor from cellular membranes, the characterization of receptor behaviour in solution and the purification of the solubilized receptor in an active form, with the extracted receptor retaining full GRP binding activity.

In particular, this invention pertains to obtaining the solubilized and purified naturally occurring GRP receptor in sequenceable grade, determining its partial amino acid sequence, isolating the cDNA for the GRP receptor, and determining the nucleotide sequence and the deduced amino acid sequence of the receptor.

Specifically, this invention pertains to expressing DNA encoding the GRP receptor in host cells, thereby enabling the synthesis of GRP receptor compositions having the amino acid sequence of the naturally occurring GRP receptor which are entirely free of other proteins of the species of origin and further enabling the synthesis of novel mutant GRP receptors.

In addition, this invention relates to the use of DNA encoding the GRP receptor or its fragments in the hybridization diagnosis of defective GRP receptor DNA or mRNA and for obtaining DNA encoding the GRP receptor from natural sources.

More specifically, this invention pertains to the use of the recombinant GRP receptor, and cell lines transfected with vectors directing the expression of the GRP receptor and membranes from such cell lines, in drug screening assays for compounds having suitable binding affinity for the GRP receptor.

Even more specifically, this invention pertains to the recombinant GRP receptor along with protein fragments of the receptor and antibodies directed thereto that may be use- ful in diagnostic assays to determine if a patient has altered levels of the gastrin releasing peptide receptor. Assays based on detection of antibodies to the GRP receptor and/or detection of the GRP receptor itself may also have prognostic value.

Additionally, this invention pertains to using the recombinant GRP receptor or fragments or derivatives thereof such as antibodies to the receptor or fragments or specific receptor antagonists defined in screening assays, as therapeutic agents.

Figure 1 is a graphical comparison of the ability of several detergents to solubilize the GRP receptor and shows the effect of solubilization on binding activity.

Figure 2 is a graph of GRP binding activity and GRP receptor solubilization as a function of detergent (CHAPS) concentration.

Figure 3 is a graph of GRP receptor solubilization and activity as a function of the soluble cholesteryl ester stabilizing agent (CHS) concentration.

Figure 4 is a graph of GRP binding activity as a function of detergent (CHAPS) concentration.

Figure 5 is a gel display of SDS-PAGE analysis of $^{125}$I-GRP cross-linked to the GRP receptor in a crude soluble extract.

Figure 6 is a silver stained gel display of SDS-PAGE analysis of the purified GRP receptor.

Figure 7 shows the separation of tryptic fragments of the GRP receptor by reverse-phase HPLC.

Figure 8 is the nucleotide sequence of the GRP receptor and its deduced amino acid sequence. The experimentally determined amino acid sequence of the intact GRP receptor protein and of isolated tryptic peptides to the receptor are indicated by underlining. Putative transmembrane sequences are labeled I through VII. Consensus sequences for N-linked glycosylation are boxed.

Figure 9 shows the hydropathy analysis of the deduced amino acid sequence of the GRP receptor. The transmembrane sequences are indicated by numbers I through VII.

Figure 10 shows the Northern hybridization analysis of mRNA from Swiss 3T3 cells.

Figure 11 is a comparison of the amino acid sequences of the GRP receptor and the Substance K receptor.

Figure 12 shows the Northern hybridization analysis of mRNA from human fetal lung cells (HFL).

Figure 13 shows the GRP ligand-dependent induction of chloride current in a Xenopus oocyte expressing an in vitro transcript from the GRP receptor cDNA clone.

This invention provides the amino acid sequence and DNA sequence of the gastrin releasing peptide (GRP) receptor which were obtained after the GRP receptor was purified and the amino acid sequence of tryptic fragments of the GRP receptor was determined.

Partial amino acid sequences obtained from the purified GRP receptor were used to deduce DNA probes which were then used to isolate the GRP receptor cDNA form of the gene. Some of the standard methods are described, or referenced in T. Maniatis et al., Molecular Cloning, A Laboratory Manual (Cold Spring Harbor Laboratory, Cold Spring Harbor, NY), or F.M. Ausubel et al., Biology (Greene Publishing Associates, Brooklyn, NY). The procedure is broadly set forth below.

A cDNA library, constructed in lambda gtlO bacteriophage, was prepared from RNA isolated from Swiss 3T3 cells. Several modifications and unique techniques had to be utilized to overcome problems associated with isolating a cDNA clone when probing the library with oligonucleotides. In particular, it was necessary to enrich the library for cDNA species encoding the GRP receptor due to the under representation of such species in unenriched cDNA libraries. Oligonucleotide probes were designed having a nucleotide sequence based upon the most likely codon usage. The cDNA library was plated out, allowing the lambda phage containing cDNA inserts to lyse their E.coli hosts and form plaques, each containing individual cDNA inserts. The plaques were screened for GRP receptor DNA sequences with labeled oligonucleotide probes. GRP receptor cDNA species were isolated, but these did not encode the complete GRP receptor.

Polymerase chain reaction technology was used to isolate additional cDNA species encoding portions of the GRP receptor, and its 5' and 3' flanking regions. Gene specific primer directed cDNA cloning was then used to obtain a single cDNA clone encoding the entire GRP receptor translation product. The actual cloning techniques utilized herein are set forth in detail in Examples 12 and 13.

Once the cDNA for the GRP receptor was isolated from mouse, it was sequenced. The nucleotide sequence revealed the amino acid sequence of the primary translation product of the GRP receptor, i.e., the amino acid sequence before any post-translational modification.

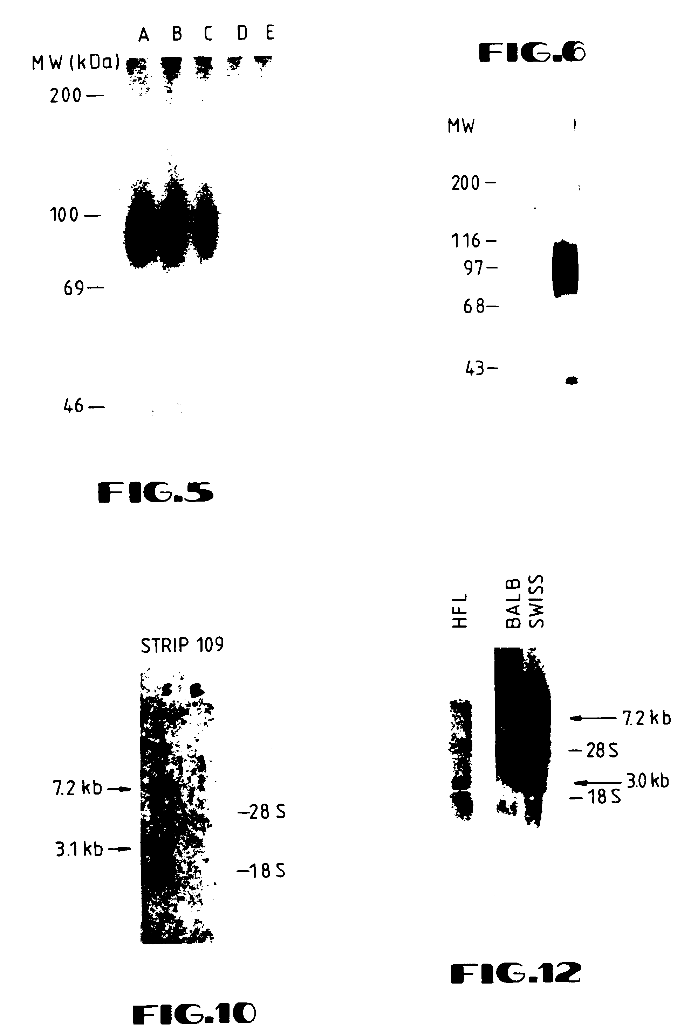

The complete amino acid sequence is shown in Figure 8. As used herein, the term "GRP receptor" shall be defined as a protein or peptide having the amino acid sequence shown in Figure 8 or a fragment thereof. The term "GRP receptor" shall also be used herein to include the GRP receptors of species other than mouse, for example humans and other mammals.

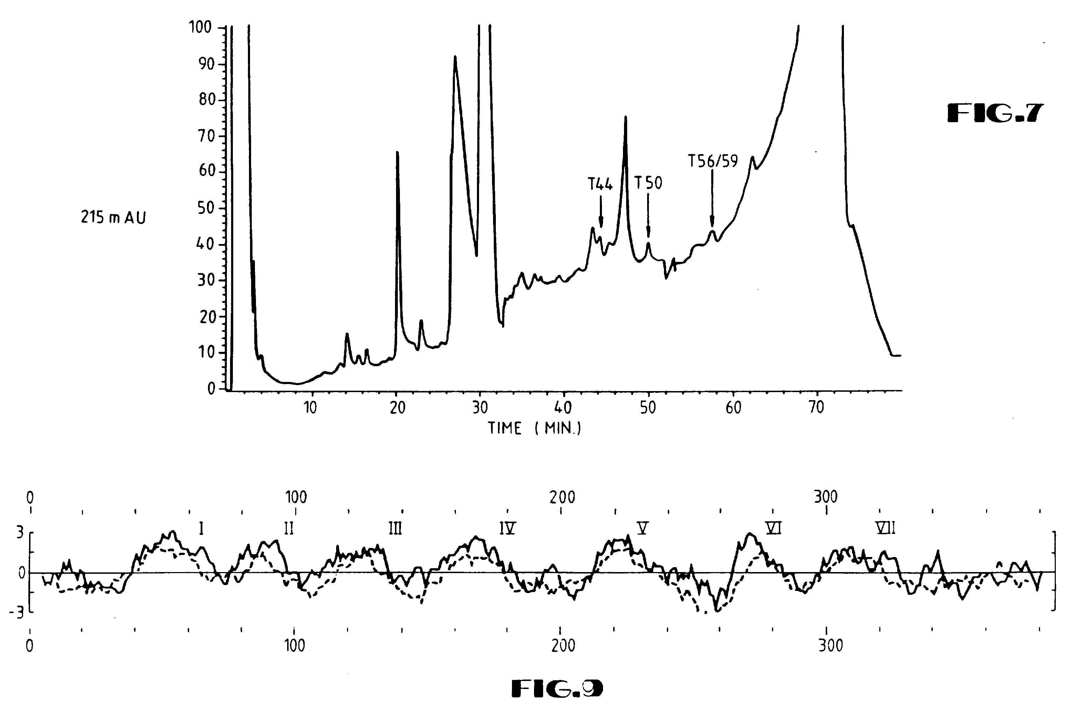

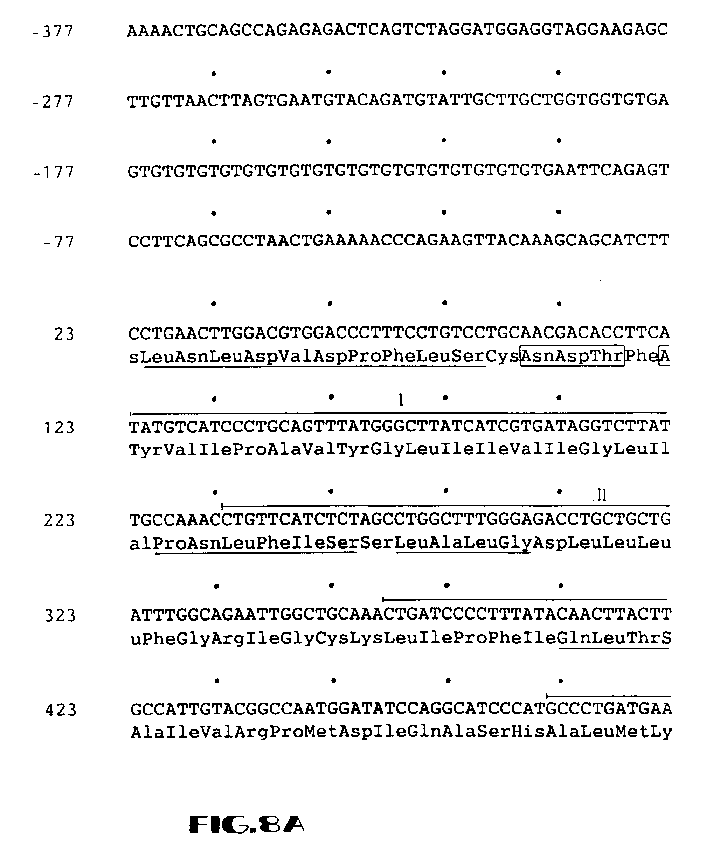

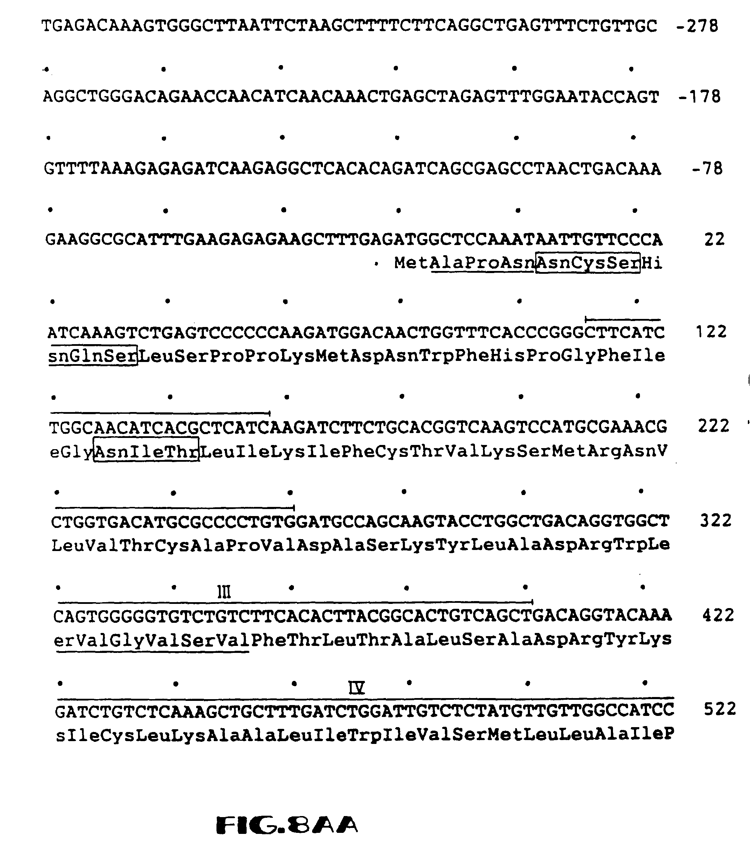

This invention also encompasses proteins or peptides having substantial homology with the amino acid sequence in Figure 8, excluding any protein or peptide which exhibits substantially the same or lesser homology, than does the Substance K receptor. This is illustrated in Figure 11.

Homology is determined by optimizing residue matches by introducing gaps as required. This changes when considering conservative substitutions as matches. This definition is intended to also include natural allelic variations in the GRP receptor sequence. Typical homologous proteins or peptides will have from 25-100% homology (if gaps can be introduced) to 50-100% homology (if conservative substitutions are included) with the amino acid sequence of Figure 8. Some homologous proteins or peptides, such as GRP receptor subtypes, will exhibit some biological activity in common with the GRP receptor of Figure 8. As used herein, the term "biological activity" is defined as including without limitation, gastrin releasing peptide binding, cross-reactivity with anti-GRP receptor antibodies raised against the GRP receptor from natural sources, and coupling to guanyl nucleotide regulatory proteins.

This invention contemplates use of the isolated DNA which encodes the GRP receptor or any fragment thereof, to encode a biologically active GRP receptor polypeptide. In addition, this invention covers isolated DNA which encodes a biologically active protein having GRP receptor activity and which is capable of hybridizing with the DNA shown in Figure 8. Said biologically active protein can be the GRP receptor itself and have the amino acid sequence shown in Figure 8. Further this invention covers the use of isolated DNA which encodes proteins which are homologous to the GRP receptor and which was isolated using GRP receptor cDNA as a probe. The isolated DNA can have the respective regulatory sequences in the 5' and 3' flanks.

It is expected that the DNA which codes for the gastrin releasing peptide receptor will be particularly useful to identify genes, mRNA and cDNA species which code for related or homologous receptors, along with those which code for GRP receptor sub-types and for the GRP receptor in tissue of different species. There is at least one GRP receptor sub-type described with a different selectivity towards bombesin-like peptides. There are likely others. Various GRP receptor sub-types are expected to be highly homologous. However, even receptor proteins that have a more distant relationship to the GRP receptor and no longer bind gastrin releasing peptide, can readily be isolated using the GRP receptor sequence if they are sufficiently homologous.

This invention further covers recombinant DNA molecules having a DNA sequence identical to the isolated DNA set forth herein.

The isolated GRP receptor DNA can be readily modified by nucleotide substitutions, nucleotide deletions, nucleotide insertions and inversions of nucleotide stretches. These modifications result in novel DNA sequences which encode the GRP receptor, its derivatives or proteins having GRP receptor activity. These modified sequences can be used to produce mutant GRP receptor or to enhance the expression of GRP receptor species. Such mutant GRP receptor derivatives include the predetermined or site-specific mutations of the GRP receptor or its fragments. Mutant GRP receptor is defined herein as being a polypeptide otherwise falling within the homology definition of the GRP receptor as set forth above, but having an amino acid sequence which differs from that of the GRP receptor as found in nature, whether by way of deletion, substitution or insertion. In particular, site specific mutant GRP receptor is defined as having greater than 50% homology with the GRP receptor of Figure 8 and as having biological activity in common with the receptor of Figure 8.

Although mutation sites are predetermined, that is not a requirement. For example, in order to optimize the performance of mutants at a given residue position, random mutagenesis can be conducted at the target codon and the expressed GRP receptor mutants can then be screened for the desired activity. Methods for making substitution mutations at predetermined sites in DNA having a known sequence, are well known in the art such as M13 primer mutagenesis.

GRP receptor mutagenesis can be conducted by making amino acid insertions or deletions. Substitutions, deletions, insertions or any subcombination may be combined to arrive at a final construct. Insertions include amino or carboxyl terminal fusions. The mutations in the DNA must not place coding sequences out of reading frames and preferably will not create complementary regions that could hybridize to produce secondary mRNA structure such as loops or hairpins.

DNA which encodes the GRP receptor or fragments thereof, can be obtained by chemical synthesis, screening cDNA libraries, or by screening genomic libraries prepared from a wide variety of cell lines or tissue samples.

This DNA can be expressed in a wide variety of host cells for the synthesis of the full-length receptor or fragments of the receptor which can in turn, for example, be used to generate polyclonal or monoclonal antibodies; for binding studies; for construction and expression of modified receptor molecules; and for structure/function studies. The GRP receptor or its fragments can be expressed in host cells that are transformed or transfected with appropriate expression vectors. These molecules can be substantially free of protein or cellular contaminants, other than those derived from the recombinant host, and therefore are particularly useful in pharmaceutical compositions when combined with a pharmaceutically acceptable carrier and/or diluent. The receptor or portions thereof, may be expressed as fusions with other proteins.

Expression vectors are self-replicating DNA or RNA constructs containing the GRP receptor gene or its fragments usually operably linked to suitable genetic control elements that are recognized in a suitable host cell. These control elements are capable of affecting expression within a suitable host. The specific type of control elements necessary to effect expression will depend upon the eventual host cell used. Generally, genetic control elements can be a procaryotic promoter system or a eucaryotic promoter expression control system, and include a transcriptional promoter, an optional operator to control the onset of transcription, transcription enhancers to elevate the level of mRNA expression, a sequence that encodes a suitable ribosome binding site, and sequences that terminate transcription and translation. Expression vectors also usually contain an origin of replication that allows the vector to replicate independently of the host cell.

The vectors of this invention contain DNA which encodes the GRP receptor or a fragment hereof encoding a biologically active GRP receptor polypeptide. The DNA can be under the control of a viral promoter and can encode a selection marker. This invention further contemplates use of such expression vectors which are capable of expressing eucaryotic cDNA coding for the GRP receptor in a procaryotic or eucar-yotic host, where the vector is compatible with the host and where the eucaryotic cDNA coding for the GRP receptor is inserted into the vector such that growth of the host containing the vector expresses the cDNA in question. Expression vectors need not always replicate in their host cells to be useful, however. Usually, expression vectors are designed for stable replication in their host cells or for amplification to greatly increase the total number of copies of the desirable gene per cell. However, it is not always necessary to require that an expression vector replicate in a host cell. However, it is possible to effect transient expression of the GRP receptor or its fragments in various hosts using vectors that do not contain a replication origin that is recognized by the host cell. It is also possible to use vectors that cause integration of GRP receptor or its fragments into the host DNA by recombination.

Vectors comprise plasmids, viruses, bacteriophage, integratable DNA fragments, and other vehicles which enable the integration of DNA fragments into the genome of the host. Expression vectors are specialized vectors which contain genetic control elements that effect expression of operably linked genes. Plasmids are the most commonly used form of vector but all other forms of vectors which serve an equiv- alent function and which are, or become, known in the art are suitable for use herein.

Transformed cells are cells, preferably mammalian, that have been transformed or transfected with GRP receptor vectors constructed using recombinant DNA techniques. Transformed host cells usually express the GRP receptor or its fragments but for purposes of cloning, amplifying, and manipulating its DNA, do not need to express the GRP receptor. This invention further contemplates culturing transformed cells in a nutrient medium, thus permitting the GRP receptor to accumulate in the culture. The GRP receptor can be recovered, first from the culture and then from the culture medium.

For purposes of this invention, DNA sequences are operably linked when they are functionally related to each other. For example, DNA for a presequence or secretory leader is operably linked to a polypeptide if it is expressed as a preprotein or participates in location of the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression.

Suitable host cells include procaryotes, lower eucaryotes and yeasts or higher eucaryotes. Procaryotes include both gram negative and gram positive organisms, for example E. coli and B. subtilis. Lower eucaryotes include yeasts, for example S. cerevisiae and Pichia, and species such as Dictyostelium. Higher eucaryotes include established tissue culture cell lines from animal cells, both of non- mammalian origin such as insect cells and of mammalian origin such as human, primates, and rodents.

Procaryotic host-vector systems include a wide variety of vectors for many different species. As used herein, E. coli and its vectors will be used generically to include equivalent vectors for other procaryotes. A representative vector for amplifying DNA is pBR322 or any of its derivatives. Vectors that can be used to express the GRP receptor or its fragments include but are not limited to such vectors as those containing the lac promoter (pUC-series); trp promoter (pBR322-trp); lpp promoter (the pIN-series); lambda-pP or pR promoters (pOTS); or hybrid promoters such as ptac (pDR540). See Brosius et al., "Expression Vectors Employing Lambda-, trp-, lac-, and lpp-derived Promoters", in Vectors: A Survey of Molecular Cloning Vectors and Their Uses, (eds. Raymond L. Rodriguez and David T. Denhardt), Buttersworth, Boston, 1988, Chapter 10, pp. 205-236.

Lower eucaryotes such as yeasts and Dictyostelium may be transformed with GRP receptor containing vectors. For purposes of this invention, the most common lower eucaryotic host is the baker's yeast, Saccharomyces cerevisiae and it will be used to generically represent lower eucaryotes although a number of other strains and species are also available. Yeast vectors consist of a replication origin (unless of the integrating type), a selection gene, a promoter, DNA encoding the GRP receptor or it fragments, sequences for translation termination, polyadenylation and transcription termination. Suitable expression vectors for yeast include such constitutive promoters as 3-phosphoglycerate kinase and various other glycolytic enzyme gene promoters or such inducible promoters as the alcohol dehydrogenase 2 promoter or metallothionine promoter. Suitable vectors include derivatives of the following types: self-replicating low copy number (such as the YRp-series), self-replicating high copy number (such as the YEp-series); integrating types (such as the YIp-series), or mini-chromosomes (such as the YCp-series).

Higher eucaryotic tissue culture cells are the preferred host cells for expression of the functionally active GRP receptor protein. In principle, any higher eucaryotic tissue culture cell line is workable, whether from an invertebrate or vertebrate source. However, mammalian cells are preferred. Transformation or transfection and propagation of such cells has become a routine procedure. Examples of useful cell lines include HeLa cells, Chinese hamster ovary (CHO) cell lines, baby rat kidney (BRK) cell lines, insect cell lines and monkey (COS) cell lines. Expression vectors for such cell lines usually include an origin of replication, a promoter, a ribosome binding site, RNA splice sites (if genomic DNA is used), a polyadenylation site, and a transcription termination site. These vectors also usually contain a selection gene or amplification gene. Suitable expression vectors may be plasmids, viruses or retroviruses carrying promoters derived from such sources as from adenovirus, SV40, parvoviruses, vaccinia virus, or cytomegalovirus. Representative examples of suitable expression vectors include pCDNA1; pCD (Okayama et al., Mol.Cell Biol. 5:1136-1142, 1985); pMClneo PolyA (Thomas et al, Cell 51:503-512, 1987); and a baculovirus vector such as pAC 373 or pAC 610.

The GRP receptor can be solubilized from membranes in an active form, and purified without loss of activity by the methods outlined below. The source of GRP receptor can be a eucaryotic or procaryotic host expressing recombinant GRP receptor, such as is described above. The source can also be a cell line such as mouse Swiss 3T3 fibroblasts, but other mammalian cell lines are also contemplated by this invention, with the preferred cell line being human.

The active GRP receptor was solubilized from membranes containing the GRP receptor using a stabilizing agent and a detergent. The stabilizing agent is preferably a soluble cholesteryl ester. Particularly good results have been obtained using cholesteryl hemisuccinate (CHS). The detergent can be non-ionic, zwitter-ionic or the like. Particularly good results have been obtained using the zwitter-ionic detergent 3-[(3-cholamidopropyl)dimethylammonio]-1- propane sulfonate (CHAPS).

Cellular membranes containing the GRP receptor are prepared by lysis of a cultured GRP receptor containing cell line such as Swiss 3T3 fibroblasts, followed by centrifugation. The resulting pellets are washed by resuspension and centrifuged again.

Once the membranes are obtained from a suitable cell line as described above and in Example 1, the final concentration of protein is adjusted. A suitable final protein concentration is about 15 mg/ml.

The membranes are then salt washed prior to solubilization of the GRP receptor. The membranes are washed twice with buffer and NaCl, then washed with a solubilization buffer and finally suspended in the solubilization buffer at an adjusted protein concentration. A suitable buffer composition for the first two washings comprises a medium such as 50 mM4-(2-hydroxyethyl)- 1-piperazine ethane sulfonic acid (HEPES), pH 7.5, a chelator such as 2 mM ethylenediaminetetraacetic acid (EDTA), and protease inhibitors. A suitable NaCl concentration is 1.0 M. The solubilization buffer, both for the washing and suspension, can be typically comprised of 50 mM HEPES, pH 7.5, 2 mM EDTA, another chelator such as 1mM [ethylenebis-(oxyethylenenitrilo)]tetraacetic acid (EGTA), 100 mM NaCl and protease inhibitors. The protein concentration is adjusted to about 7 mg/ml, for example. This salt washing step provides a 2 fold purification. Similar results can be achieved by washing the membranes with 2 M urea, high pH buffers (pH 10) or chaotropic salts such as KI. This procedure also increases the stability of the GRP receptor in the extract. Other constituents of the buffers may include, for example, sucrose, and suitable protease inhibitors include, without limitation, aprotinin, leupeptin, pepstatin, bacitrin and phenylmethylsulfonyl fluoride (PMSF).

A mixture of detergent (CHAPS) and soluble cholesteryl ester stabilizing agent (CHS) is then slowly added to the membrane suspension to give a set final detergent concentration. The weight ratio of detergent to soluble cholesteryl ester can be within the range of about 200:1 to 5:2, preferably about 10:1. Alternatively, the detergent can be added to the membrane suspension, followed by the addition of the soluble cholesteryl ester. In that instance, initially there will be 100% detergent and the soluble cholesteryl ester is added until the weight ratio of detergent to ester is within the range of about 200:1 to 5:2, preferably about 10:1. For solubilization of the GRP receptor, the concentration of detergent should be 0.4 to 3.0% (w/v), and is optimally set at about 0.75% (w/v) for a membrane concentration (prior to the membrane washing steps) of around 15 mg/ml. Similarly, the concentration of soluble cholesteryl ester is within the range of about 0.0015% to 1.2% (w/v). Likewise, for a membrane concentration of around 15 mg/ml, the concentration of soluble cholesteryl ester is preferably about 0.075% (w/v).

The extract is then incubated at a temperature within the range of about 0 to 37°C, typically room temperature such as 21°C, and then cooled to 0 to 21°C, typically 4°C, and the insoluble material centrifuged at high speeds, preferably about 100,000 times gravity, in a standard centrifuge for a suitable period of time depending upon the volume involved to obtain an extract containing the solubilized receptor (i.e., soluble extract).

At the high detergent concentration (0.4 to 3.0%), the receptor is not active. However, upon dilution with a buffer solution, the receptor is reactivated. The presence of the soluble cholesteryl ester, which acts as a stabilizing agent, is necessary for the receptor to be reactivated at the low detergent concentration. For assays using the active solubilized GRP receptor, to exhibit binding activity the final concentration of detergent in the suspension should be diluted to within the range of about 0.025 to 0.2% (w/v). The weight ratio of detergent to soluble cholesteryl ester is still maintained within the range of about 200:1 to 5:2, preferably about 10:1. Therefore, a suitable range for the soluble cholesteryl ester is about 0.000125% to 0.08% (w/v). The preferable assay concentrations are 0.075% (w/v) detergent and about 0.0075% (w/v) soluble cholesteryl ester.

The solubilized receptor in its active form is then purified and freed of contaminating proteins. Purification of the GRP receptor involves a multistep procedure which includes the following steps, which follow the solubilization procedure set forth above.

(1) Polyethylene glycol precipitation. The GRP receptor is precipitated from the soluble extract by addition of polyethylene glycol (PEG). Addition of PEG is preferably done to obtain a final concentration of 20% (w/v). The precipitate is then collected by centrifugation and resuspended in a buffer solution. The buffer solution can typically be comprised of 25 mM HEPES, pH 7.5, 25 mM Tris/Cl, 2 mM EDTA, 0.075% (w/v) detergent, 0.0075% (w/v) soluble cholesteryl ester, and protease inhibitors. The final volume of the suspension is preferably 25% that of the original soluble extract. Proteins remaining insoluble in the suspension are removed by centrifugation. This step provides a 2 fold purification, and enhances the stability of the receptor.

(2) Wheat germ agglutinin chromatography. The soluble extract is applied to a wheat germ agglutinin affinity column equilibrated with a buffer solution typically comprised of 50 mM HEPES, pH 7.5, 2 mM EDTA, 0.25% (w/v) detergent, 0.025% (w/v) cholesteryl ester and protease inhibitors. The column is eluted with column buffer solution and 5 mM N-N'-N"triacetyl-chitotriose. Fractions containing the GRP receptor are then identified by $^{125}$I-GRP binding assays. This step provides a 5 fold purification by removing proteins that do not contain carbohydrate. To obtain a good yield, it is necessary to elute the column with chitotriose or chitobiose. The yield may also be enhanced by maintaining the detergent concentration above about 0.2% detergent and 0.02% soluble cholesteryl ester.

(3) GRP-affinity chromatography. The wheat germ agglutinin column eluate is further fractionated on a GRP affinity column. In the preferred embodiment, the column contains a beaded matrix with the peptide human [Nle14,27] GRP13-27 (the C-terminal portion of GRP) coupled to it at 2 mg peptide/ml packed gel. The column is equilibrated with a solution typically comprised of 25 mM Tris, 25 mM HEPES, pH 7.5, 2 mM EDTA, 0.075% (w/v) CHAPS, 0.0075% (w/v) CHS and protease inhibitors. The concentration of dete-gent in the wheat germ agglutinin column eluate is preferably adjusted to 0.075% (w/v) by dilution with a solution typically comprised of 25 mM HEPES, 25 mM Tris, pH 7.5, 2 mM EDTA and protease inhibitors. After application of the sample and extensive washing of the column, bound protein is eluted with a salt at a concentration above 0.2 M. Particularly suitable is 0.5 M NaCl. Fractions containing the GRP receptor are then identified by $^{125}$I-GRP binding assays. The GRP peptide used ([Nle14,27]GM13-27) is an analog made by Triton Biosciences Inc. (Alameda, CA) which is resistant to oxidation. Other GRP peptides and matrixes that will also work include, without limitation, GRP1-27, GRP14-27 and [Lys3] Bombesin. However the yield and elution conditions may be altered. Elution of the bound protein with salt is important because receptor binding activity is preserved and a good yield is achieved. The concentration of detergent in the sample loaded onto the column is critical for optimal results. The suitable range of detergent is about 0.025% to 0.2% (w/v). The ratio of detergent to stabilizing agent is also the same, being 200:1 to 5:2, preferably 10:1.

(4) Second affinity column. Fractions containing the GRP receptor eluted from the affinity column are desalted and the sample is applied to a second GRP affinity column, and eluted as described in step (3). Fractions containing the receptor are then identified by binding assays. Use of two consecutive affinity columns in this step is required to give a high degree of purity.

(5) Gel filtration. This is an optional step that yields a marginally purer product. The gel filtration step is also useful to remove protease inhibitors, salt and residual detergent from the receptor.

In general, the solubilized, unpurified and solubilized, purified GRP receptor of this invention binds gastrin releasing peptide with an affinity of at least $K_D$=10 nM. The GRP receptor from a mouse Swiss 3T3 fibroblast cell line, according to this invention was found to have the following characteristics:

runs as a broad band on SDS-PAGE with an apparent molecular weight of about 70 to 100 kilodaltons; binds selectively with polypeptides of the bombesin type;
has a $K_D$ value of about 10-100 pM; is free of coupled G proteins; contains N-linked carbohydrates; when deglycosylated, has an apparent molecular weight of 36±5 kilodaltons on SDS-PAGE; and has a partial amino acid sequence near the N-terminus of:

**-Leu-Asn-Leu-Asp-Val-Asp-Pro-Phe-Leu-Ser-**

Now that the sequence is known, the GRP receptor or any fragments thereof can be prepared by conventional processes for synthesizing peptides. These include processes such as are described in John M. Stewart and Janis D. Young, Solid Phase Peptide Synthesis (Pierce Chemical Co., Rockford, IL 1984), M. Bodanszky and A.Bodanszky, The Practice of Peptide Synthesis (Springer-Verlag, New York, 1984) and M. Bodanszky, The Principles of Peptide Synthesis (Springer-Verlag, New York, 1984). For example, an azide process, an acid chloride process, an acid anhydride process, a mixed anhydride process, an active ester process (for example, p-nitrophenyl ester, N- hydroxysuccinimide ester, or cyanomethyl ester), a carbodiimidazole process, an oxidative-reductive process, or a DCC/additive process can be used. Solid phase and solution phase syntheses are both applicable to the foregoing processes.

The GRP receptor is suitably prepared in accordance with the above processes as typically employed in peptide synthesis, generally either by a so-called stepwise process which comprises condensing an amino acid to the terminal amino acid, one by one in sequence, or by coupling peptide fragments to the terminal amino acid. Amino groups that are not being used in the coupling reaction must be protected to prevent coupling at an incorrect location.

If a solid phase synthesis is adopted, the C-terminal amino acid is bound to an insoluble carrier or support through its carboxyl group. The insoluble carrier is not particularly limited as long as it has a binding capability to a reactive carboxyl group. Examples of such insoluble carriers include halomethyl resins, such as chloromethyl resin or bromomethyl resin, hydroxymethyl resins, phenol resins, tert-alkyloxycarbonyl- hydrazidated resins, and the like.

An amino group-protected amino acid is bound in sequence through condensation of its activated carboxyl group and the reactiveamino group of the previously formed peptide or chain, to synthesize the peptide step by step. After synthesizing the complete sequence, the peptide is split off from the insoluble carrier to produce the peptide. This solid-phase approach is generally described by Merrifield et al. in J. Am. Chem. Soc. 85:2149-2156 (1963).

The prepared receptor and fragments thereof can be isolated and purified from the reaction mixture by means of peptide separation, for example, by extraction, precipitation, electrophoresis and various forms of chromatography, and the like. The receptor of this invention can be obtained in varying degrees of purity depending upon its desired use. Purification can be accomplished by use of the protein purification techniques disclosed herein or by the use of the antibodies herein described in immunoabsorbant affinity chromatography. This immunoabsorbant affinity chromatography is carried out by first linking the antibodies to a solid support and then contacting the linked antibodies with solubilized lysates of small cell lung cancer cells, lysates of other cells expressing the GRP receptor, or lysates or supernatants of cells producing the GRP receptor as a result of DNA techniques described below.

Derivatives of the GRP receptor included herein include amino acid sequence mutants, glycosylation variants and covalent or aggregative conjugates with other chemical moieties. Covalent derivatives can be prepared by linkage of functionalities to groups which are found in the GRP receptor amino acid side chains or at the N- or C-termini, by means which are well known in the art. These derivatives can include, without limitation, aliphatic esters or amides of the carboxyl terminus or residues containing carboxyl side chains, O-acyl derivatives of hydroxyl group-containing residues, and N-acyl derivatives of the amino terminal amino acid or aminogroup containing residues, for example, lysine or arginine. Acyl groups are selected from the group of alkyl-moieties including C3 to C18 normal alkyl, thereby forming alkanoyl species.

A major group of derivatives are covalent conjugates of the GRP receptor or fragments thereof with other proteins of polypeptides. These derivatives can be synthesized in recombinant culture such as N- or C-terminal fusions or by the use of agents known in the art for their usefulness in crosslinking proteins through reactive side groups. Preferred GRP derivatization sites with cross-linking agents are at free amino groups, carbohydrate moieties and cysteine residues.

This invention also contemplates the use of derivatives of the GRP receptor other than variations in amino acid sequence or glycosylation. Such derivatives are characterized by covalent or aggregative association with chemical moieties. The derivatives generally fall into three classes: (1) salts, (2) side chain and terminal residue covalent modifications, and (3)adsorption complexes, for example with cell membranes. Such covalent or aggregative derivatives are useful as immunogens, as reagents in immunoassays or in purification methods such as for affinity purification of gastrin releasing peptide or other binding ligands. For example, the GRP receptor can be immobilized by covalent bonding to a solid support such as cyanogen bromide-activated Sepharose, by methods which are well known in the art, or adsorbed onto polyolefin surfaces, with or without glutaraldehyde cross-linking, for use in the assay or purification of anti-GRP receptor antibodies or gastrin releasing peptide. The GRP receptor can also be labeled with a detectable group, for example radioiodinated by the chloramine T procedure, covalently bound to rare earth chelates or conjugated to another fluorescent moiety for use in diagnostic assays.

The solubilized GRP receptor of this invention can be used as an immunogen for the production of antisera or antibodies specific for the receptor or any fragments thereof. The purified receptor can be used to screen monoclonal antibodies prepared by immunization with various forms of impure preparations containing the GRP receptor. The purified receptor can also be used as a reagent to detect any antibodies generated in response to the presence of elevated levels of gastrin releasing peptide receptor or cell fragments containing the GRP receptor. Additionally, GRP receptor fragments may also serve as immunogens to produce the antibodies of the present invention. For example, this invention contemplates antibodies having binding affinity to or being raised against the amino acid sequence shown in Figure 8, or any fragment thereof. In particular, this invention contemplates antibodies having binding affinity to or being raised against specific fragments which are predicted to lie outside of the lipid bilayer. These fragments include the following ten amino acid sequence (residues 9-18, inclusive) near the N-terminus:

<pre>
        9                                                            18
  -Leu-Asn-Leu-Asp-Val-Asp-Pro-Phe-Leu-Ser-
</pre>

In addition, as noted above, this invention covers fragments of the GRP receptor which are predicted to reside on the extracellular side of the membrane: residues 1-39, inclusive; residues 98-115, inclusive; residues 176-209, inclusive; and residues 288-300, inclusive; and to the following portions of the receptor which are predicted to reside on the intracellular side of the membrane: residues 64-77, inclusive; residues 138-157, inclusive; residues 236-266, inclusive; and residues 330-385, inclusive.

Antibodies can be raised to the GRP receptor, and fragments thereof, both in its naturally occurring form and in its recombinant form. Additionally, antibodies can be raised to both the GRP receptor in its active form and in its inactive form, the difference being that antibodies to the active receptor are more likely to recognize epitopes which are only present in the active receptor.

Antibodies against predetermined fragments of the GRP receptor can be raised by immunization of animals with conjugates of the fragments with immunogenic proteins. Monoclonal antibodies are prepared from cells secreting the desired antibody. These antibodies can be screened for binding to normal or defective GRP receptors, or screened for agonistic or antagonistic GRP receptor activity.

The antibodies of this invention can have significant therapeutic value. They can be potent antagonists that bind to the GRP receptor and inhibit ligand binding to the receptor or inhibit the ability of gastrin releasing peptide to elicit a biological response. They also can be useful as non-neutralizing antibodies and can be coupled to toxins or radionuclides so that when the antibody binds to the receptor, the cell itself is killed. Further, these antibodies can be conjugated to drugs or other therapeutic agents, either directly or indirectly by means of a linker.

The antibodies of this invention can also be useful in diagnostics. As capture or non-neutralizing antibodies, they can bind to the GRP receptor without inhibiting ligand binding. As neutralizing antibodies, they can be useful in competitive binding assays.

Receptor fragments may be joined to other materials, particularly polypeptides, as fused or covalently joined polypeptides to be used as immunogens. The GRP receptor and its fragments may be fused or covalently linked to a variety of immunogens, such as keyhole limpet hemocyanin, bovine serum albumin, tetanus toxoid, etc. See for example, Microbiology, Hoeber Medical Division (Harper and Row, 1969), Landsteiner, Specificity of Serological Reactions (Dover Publications, New York, 1962) and Williams et al., Methods in Immunology and Immunochemistry, Vol. 1 (Academic Press, New York, 1967), for descriptions of methods of preparing polyclonal antisera. A typical method involves hyperimmunization of an animal with an antigen. The blood of the animal is then collected shortly after the repeated immunizations and the gamma globulin is isolated.

In some instances, it is desirable to prepare monoclonal antibodies from various mammalian hosts, such as mice, rodents, primates, humans, etc. Description of techniques for preparing such monoclonal antibodies may be found in, Stites et al., editors, Basic and Clinical Immunology, (Lange Medical Publications, Los Altos, CA, Fourth edition) and references cited therein, and in particular in Kohler and Milstein in Nature 256: 495-497 (1975), which discusses one method of generating monoclonal antibodies. Summarized briefly, this method involves injecting an animal with an immunogen. The animal is then sacrificed and cells taken from its spleen, which are then fused with myeloma cells. The result is a hybrid cell or "hybridoma" that is capable of reproducing in vitro. The population of hybridomas is then screened to isolate individual clones, each of which secrete a single antibody species to the immunogen. In this manner, the individual antibody species obtained are the products of single B cells from the immune animal generated in response to a specific site recognized on the immunogenic substance.

The antibodies of this invention can also be used for affinity chromatography in isolating the receptor. Columns can be prepared where the antibodies are linked to a solid support, e.g., particles, such as agarose, Sephadex, or the like, where a cell lysate may be passed through the column, the column washed, followed by increasing concentrations of a mild denaturant, whereby the purified receptor protein will be released.

Both the naturally occurring and the recombinant form of the GRP receptor of this invention are particularly useful in kits and assay methods which are capable of screening compounds for binding activity to the GRP receptor. Several methods of automating assays have been developed in recent years so as to permit screening of tens of thousands of compounds per year. The development of suitable assays can be greatly facilitated by the availability of large amounts of purified, soluble receptor in an active state such as is attainable by the process of this invention.

A kit for determining the binding affinity of a test compound to the gastrin releasing peptide receptor would typically comprise a test compound; a labeled compound, for example a ligand or antibody having known binding affinity for the gastrin releasing peptide receptor; a source of gastrin releasing peptide receptor (naturally occurring or recombinant); and a means for separating bound from free labeled compound, such as a solid phase for immobilizing the gastrin releasing peptide receptor.

Once compounds are screened, those having suitable binding affinity to the GRP receptor can be evaluated in suitable biological assays, as are well known in the art, to determine whether they act as agonists or antagonists.

This invention is particularly useful for screening compounds by using the recombinant GRP receptor in any of a variety of drug screening techniques. The advantages of using the recombinant GRP receptor in screening for GRP receptor reactive drugs include: (a) improved renewable source of the receptor from a specific source; (b) potentially greater number of receptors per cell giving higher signal to noise ratio in assays; and (c) receptor subtype specificity (theoretically giving greater biological and disease specificity).

One method of drug screening utilizes eucaryotic or procaryotic host cells which are stably transformed with recombinant DNA molecules expressing the GRP receptor. Such cells, either in viable or fixed form, can be used for standard receptor/ligand binding assays. Competitive assays are particularly useful, where the cells (source of GRP receptor) are contacted and incubated with a labeled ligand having known binding affinity to the GRP receptor, such as $^{125}I$-GRP, and a test compound whose binding affinity to the GRP receptor is being measured. The bound ligand and free ligand are then separated to assess the degree of ligand binding. The amount of test compound bound is inversely proportional to the amount of labeled ligand binding measured. Any one of numerous techniques can be used to separate bound from free ligand to assess the degree of ligand binding. This separation step could typically involve a procedure such as adhesion to filters followed by washing, adhesion to plastic followed by washing, or centrifugation of the cell membranes. Viable cells could also be used to screen for the effects of drugs on GRP receptor mediated functions, for example, second messenger levels (Ca), proliferation, etc.

Another method utilizes membranes from transformed eucaryotic or procaryotic host cells as the source of the GRP receptor. These cells are stably transformed with DNA vectors directing the expression of the GRP receptor. Essentially, the membranes would be prepared from the cells and used in any receptor/ ligand binding assay such as the competitive assay set forth above.

Still another approach is to use solubilized, unpurified or solubilized, purified receptors from transformed eucaryotic or procaryotic host cells. This allows for a real "molecular" binding assay with the advantages of increased specificity, the ability to automate, and high drug test throughput.

Another technique for drug screening involves an approach which provides high throughput screening for compounds having suitable binding affinity to the gastrin releasing peptide receptor and is described in detail in Geysen, WO-A-84/03564. First, large numbers of different small peptide test compounds are synthesized on a solid substrate such as plastic pins or some other surface. Then all the pins are reacted with solubilized, unpurified or solubilized, purified GRP receptor and washed. The next step involves detecting bound GRP receptor.

Purified GRP receptor can be coated directly onto plates for use in the aforementioned drug screening techniques. However, non-neutralizing antibodies to the GRP receptor can be used as capture antibodies to immobilize the GRP receptor on the solid phase.

This invention also contemplates the use of competitive drug screening assays where neutralizing antibodies to the receptor or receptor fragments compete with a test compound for binding to the receptor. In this manner, the antibodies can be used to detect the presence of any polypeptide which shares one or more binding sites of the GRP receptor and can also be used to occupy binding sites on the receptor that might otherwise be occupied by gastrin releasing peptide.

Additionally, neutralizing antibodies against the receptor and soluble fragments of the receptor which contain the high affinity ligand binding site, can be used to inhibit gastrin releasing peptide receptor function in cancerous tissues.

This invention also contemplates use of the GRP receptor, fragments thereof, peptides, and their fusion products in a variety of diagnostic kits and methods for detecting the presence of the gastrin releasing peptide receptor.

A kit for determining the concentration of gastrin releasing peptide receptor in a sample would typically comprise a labeled compound (ligand or antibody) having known binding affinity for the gastrin releasing peptide receptor, a source of gastrin releasing peptide receptor (naturally occurring or recombinant) and a means for separating the bound from free labeled compound, for example a solid phase for immobilizing the gastrin releasing peptide receptor.

A method for determining the concentration of gastrin releasing peptide receptor in a sample would typically comprise the steps of: (1) preparing membranes from a sample comprised of a GRP receptor source; (2) washing the membranes and suspending them in a buffer; (3) solubilizing the GRP receptor by incubating the membranes in a culture medium to which a detergent and a soluble cholesteryl ester has been added; (4) adjusting the detergent concentration of the solubilized receptor; (5) contacting and incubating said dilution with radiolabeled GRP to form GRP:GRP receptor complexes; (6) recovering the complexes such as by filtration through polyethyleneimine treated filters; and (7) measuring the radioactivity of the recovered complexes.

Antibodies specific for the receptor or receptor fragments are useful in diagnostics to detect the presence of elevated levels of the receptor and/or its fragments. Such diagnostic assays can employ lysates, fixed cells, immunofluorescence, and further can involve the detection of antigens related to the GRP receptor in serum, or the like. Diagnostic assays may be homogeneous (without a separation step between free reagent and receptor-ligand complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substratelabeled fluorescent immunoassay (SLFIA) and the like. For example, unlabeled antibodies can be employed by using a second antibody which is labeled and which recognizes the antibody to the GRP receptor or to a particular fragment thereof. These assays have also been extensively discussed in the literature.

Frequently, the reagents for diagnostic assays are supplied in kits, so as to optimize the sensitivity of the assay. For the subject invention, depending upon the nature of the assay, the protocol, and the label, either labeled or unlabeled antibody, or labeled receptor is provided, usually in conjunction with other additives, such as buffers, stabilizers, materials necessary for signal production such as substrates for enzymes, and the like. Desirably, the reagents are provided as a dry powder, where the reagents may be reconstituted in an aqueous medium having appropriate concentrations for performing the assay.

Any of the aforementioned constituents of the drug screening and the diagnostic assays may be used without modification or may be modified in a variety of ways. For example, by labeling such as covalently or non-covalently joining a moiety which directly or indirectly provides a detectable signal. In any of these assays, the ligand, test compound, GRP receptor, or antibodies thereto can be labeled either directly or indirectly. Possibilities for direct labeling include label groups which include: radiolabels such as 1251, enzymes (U.S.Pat.No. 3,645,090) such as peroxidase and alkaline phosphatase, and fluorescent labels (U.S.Pat.No. 3,940,475) capable of monitoring the change in fluorescence intensity, wavelength shift, or fluorescence polarization. Possibilities for indirect labeling include biotinylation of one constituent followed by binding to avidin coupled to one of the above label groups.

There are also numerous methods of separating the bound from the free ligand, or alternatively the bound from the free test compound. The receptor can be immobilized on various matrixes followed by washing. Suitable matrixes include plastic such as an ELISA plate, filters, and beads. Methods of immobilizing the receptor to a matrix include direct adhesion to plastic, use of a capture antibody, chemical coupling, and biotin-avidin. The last step in this approach involves the precipitation of receptor/ligand complex by any of several methods including those utilizing an organic solvent such as polyethylene glycol or a salt such as ammonium sulfate. Other suitable separation techniques include, without limitation, the fluorescein antibody magnetizable particle method described in S.J. Rattle et al., Clin.Chem. 30 (9): 1457-1461 (1984) and the double antibody magnetic particle separation as described in U.S.Pat.No. 4,659,678.

The methods for linking protein receptors or their fragments to the various labels have been extensively reported in the literature and do not require detailed discussion here. Many of the techniques involve the use of activated carboxyl groups either through the use of carbodiimide or active esters to form peptide bonds, the formation of thioethers by reaction of a mercapto group with an activated halogen such as chloroacetyl, or an activated olefin such as maleimide, for linkage, or the like.

Another diagnostic aspect of this invention involves use of oligonucleotide and polynucleotide sequences taken from the GRP receptor sequence which can be used as probes for detecting levels of the gastrin releasing peptide receptor in patients suspected of having cancer. The preparation of both RNA and DNA nucleotide sequences, the labeling of the sequences and the preferred size of the sequences has received ample description and discussion in the literature. Normally an oligonucleotide probe should have at least about 14 nucleotides, usually at least about 18 nucleotides, and the polynucleotide probes may be up to several kilobases. Various labels may be employed, most commonly radionuclides, particularly 32P. However, other techniques may also be employed, such as using biotin modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed which can recognize specific duplexes, including DNA duplexes, RNA duplexes, DNA-RNA hybrid duplexes, or DNA-protein duplexes. The antibodies in turn may be labeled and the assay carried out where the duplex is bound to a surface, so that upon the formation of duplex on the surface, the presence of antibody bound to the duplex can be detected. The use of probes to the novel anti-sense RNA may be carried out in any conventional techniques such as nucleic acid hybridization, plus and minus screening, recombinational probing, hybrid released translation (HRT) and hybrid arrested translation (HART). This also includes amplification techniques such as polymerase chain reaction (PCR).

This invention has significant therapeutic value. It is expected that the GRP receptor (naturally occurring or recombinant), fragments thereof and antibodies thereto, along with compounds identified as having binding affinity to the GRP receptor, will be useful in the treatment of cancerous tissues such as prostatic and pancreatic tumors, and in the treatment of small cell lung cancer. Additionally, it is further believed that this invention will have therapeutic value in any disease or disorder associated with abnormal expression or abnormal triggering of the GRP receptor. For example, it is believed that the GRP receptor plays a role in neurologic function, and can affect gastrointestinal, pulmonary, and brain tissue.

Recombinant GRP receptor or GRP receptor antibodies can be purified and then combined for therapeutic use with conventional pharmaceutically acceptable carriers or diluents, alone with physiologically innocuous stabilizers and excipients. These combinations can then be sterile filtered and placed into dosage forms as by lyophilization in dosage vials or storage in stabilized aqueous preparations. This invention also contemplates use of antibodies which are not complement binding.

Drug screening using the GRP receptor or fragments thereof can be done to identify compounds having binding affinity to the GRP receptor. Subsequent biological assays can then be utilized to determine if the compound has intrinsic stimulating activity and is therefore a blocker or antagonist in that it blocks the activity of gastrin releasing peptide. Likewise, a compound having intrinsic stimulating activity can activate the receptor and is thus an agonist in that it simulates the activity of gastrin releasing peptide. This invention further contemplates the therapeutic use of antibodies to the GRP receptor as antagonists.

The GRP receptor (recombinant), fragments thereof, and antibodies to the receptor or its fragments, antagonists, and agonists, may be administered directly to the host to be treated or depending on the size of the compounds, it may be desirable to conjugate them to carrier proteins such as ovalbumin or serum albumin prior to their administration. Therapeutic formulations may be administered in any conventional dosage formulation. While it is possible for the active ingredient to be administered alone, it is preferable to present it as a pharmaceutical formulation. Formulations comprise at least one active ingredient as defined above together with one or more acceptable carriers thereof. Each carrier must be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. The therapy of this invention may be combined with or used in association with other chemotherapeutic or chemopreventive agents.

The broad scope of this invention is best understood with reference to the following examples, which are not intended to limit the inventions in any manner.

EXAMPLE 1

Preparation of Mouse 3T3 Fibroblast Membranes

Mouse Swiss 3T3 fibroblasts were grown to confluence in Dublecco's modified Eagles medium supplemented with 10% (vol/vol) fetal calf serum in T-850 roller bottles (lots of 100) at 37°C in a 10% $CO_2$/90% air environment. Upon harvest, the medium was poured off and each bottle was rinsed twice with 50 ml calcium/magnesium free phosphate buffered saline (PBS-CMF). Cells were incubated with 25-30 ml 0.04% (wt/vol) EDTA in PBS-CMF (warmed to 37°C) for 15 minutes at room temperature. The cells were then removed with firm knocks and pipetted into conical 250 ml centrifuge tubes on ice. Cells from six roller bottles were combined into each centrifuge tube. Roller bottles were rinsed a final time with 25 ml PBSCMF. Cells were pelleted at 1800 rpm for 10 minutes at 4°C in a Sorvall RC-3B centrifuge. Each pellet was resuspended in 50 ml fresh PBS-CMF at 4°C. Cells from 2-3 centrifuge tubes were combined, pelleted and washed with an additional 120 ml PBSCMF. The final cell pellets were resuspended in 200 ml lysis buffer (50 mM HEPES, pH 7.5, 2 mM $MgCl_2$, 1 mM EGTA, 50 µg/ml leupeptin, 2.5 µg/ml pepstatin, 10 µg/ml aprotinin and 0.5 mM phenylmethylsulfonyl fluoride (PMSF)). Cells were lysed by $N_2$ cavitation. Briefly, 100 ml of the cell suspension was placed in ice in a sealed stainless steel container which was pressurized to 900 psi of $N_2$. The suspension was slowly released from the chamber through a small orifice into a collection tube, causing rapid decompression and lysis of the cells. Cell lysis appeared complete by microscopic visualization. Membranes were pelleted at 39,000 x g for 30 minutes at 4°C, resuspended in lysis buffer and pelleted again. The pellet was suspended at a concentration of 15 mg membrane protein/ml in a storage buffer (50 mM HEPES, pH 7.5, 1 mM EGTA, 0.25 M sucrose, 50 µg/ml leupeptin, 2.5 µg/ml pepstatin, 10 µg/ml aprotinin and 0.5 mM PMSF). Membranes were aliquoted in volumes of 1 and 5 ml, flash-frozen in liquid $N_2$, and stored at -80°C.

## EXAMPLE 2

### Comparison of Detergents for Solubilization of the GRP Receptor

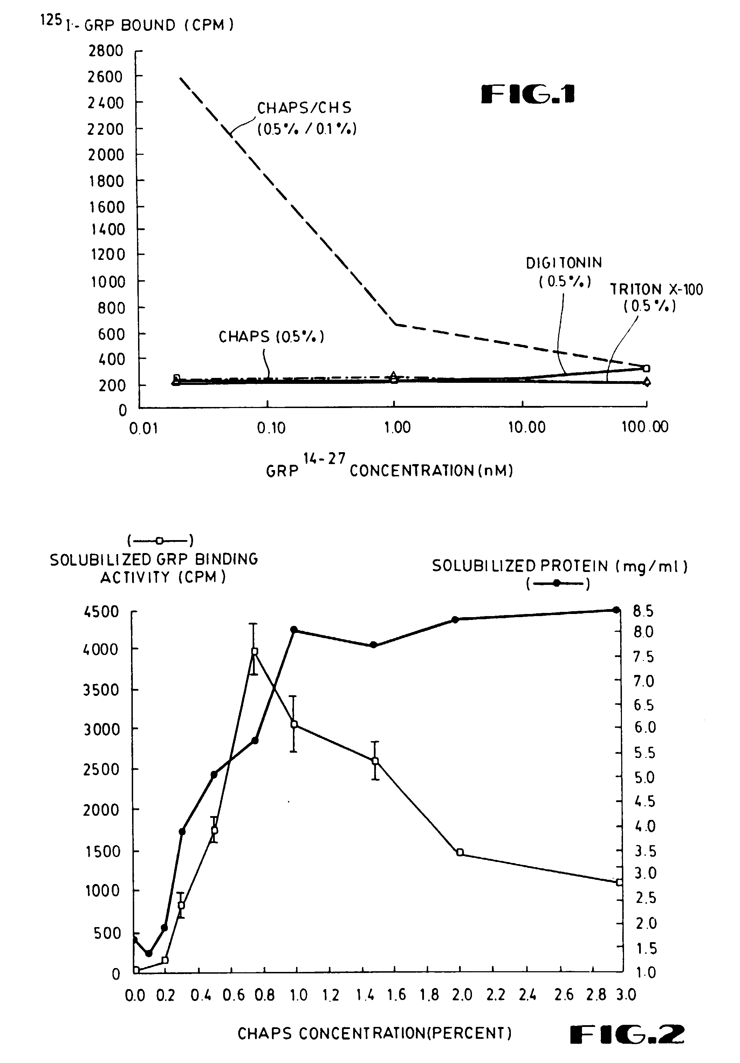

Several detergents employed for receptor extraction in other systems were tested to measure their ability to solubilize GRP receptor from Swiss 3T3 fibroblast membranes. Digitonin, Triton X-100, CHAPS, and CHAPS with CHS were all used to extract membranes at a detergent concentration of 0.50% and all were effective in solubilizing receptor that had been radio-labeled by cross-linking to $^{125}$I-GRP. The binding of $^{125}$I-GRP (0.02 nM), measured as counts/minute (CPM) bound, was assayed in the presence of the detergent (0.1%) used in the extraction and several concentrations of the unlabeled 14-27 C- terminal amino acids of GRP (GRP14-27), as is shown in Figure 1. Only extraction with CHAPS plus CHS yielded detectable binding activity. Since all detergents were effective in solubilizing the GRP receptor, the failure to observe binding activity in extracts prepared with digitonin, Triton X-100 and CHAPS, was a result of receptor inactivation during the solubilization process. It was noted however, that partial reactivation of the receptor extracted with CHAPS (without CHS) could be achieved by subsequent addition of CHS. This established that CHS acts as a stabilizer in promoting the active GRP receptor.

### Comparison of Detergent Concentration for Solubilization of the GRP Receptor

Swiss 3T3 fibroblast membranes, prepared as in Example 1, were incubated with various concentrations of the detergent CHAPS. After separation of insoluble material by centrifugation, soluble GRP binding activity was measured in the supernatant. When 0.75% (w/v) CHAPS was used to solubilize the GRP receptor, maximal receptor binding was observed, as is shown in Figure 2. However, to obtain maximal solubilization of protein, CHAPS concentration of 1.0% (w/v) or greater was required. The GRP receptor binding declined steadily at higher detergent concentrations. In order to observe specific GRP binding to receptors solubilized by CHAPS, it was necessary to include the stabilizing agent CHS. The ratio of CHAPS:CHS was maintained at 10:1 under both extraction and assay conditions.

### Comparison of Stabilizing Agent Concentration for Solubilization of the GRP Receptor

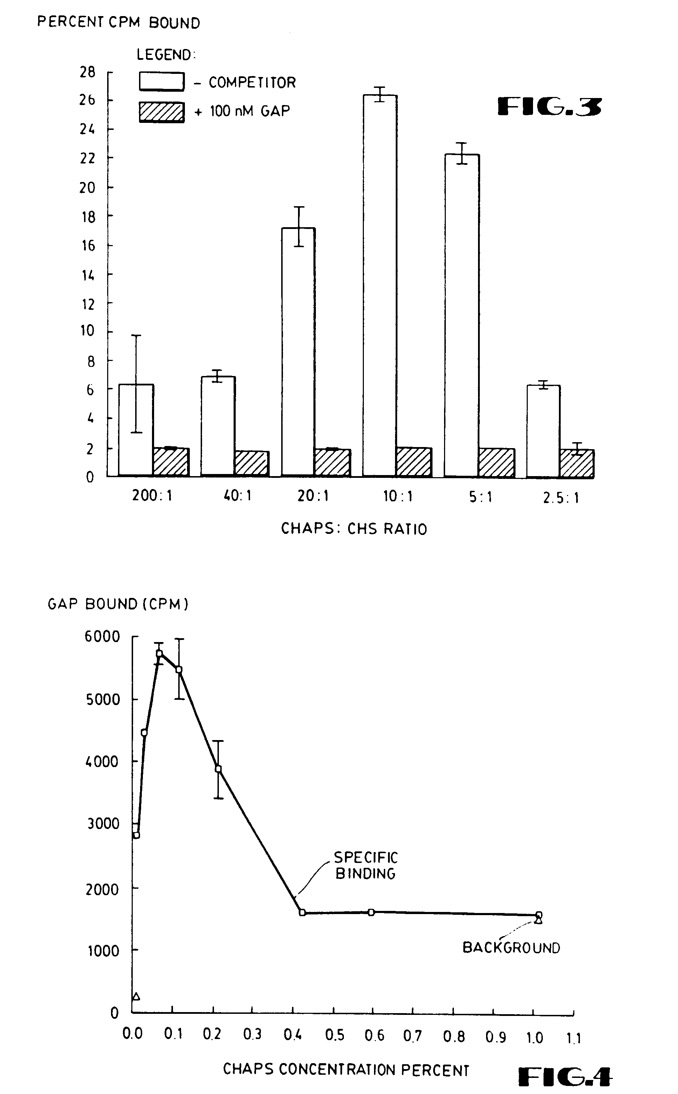

Swiss 3T3 fibroblast membranes, prepared as in Example 1, were solubilized with 0.75% (w/v) CHAPS in the presence of various amounts of cholesteryl hemisuccinate (CHS). After the removal of insoluble material by centrifugation, soluble GRP receptor binding activity was measured in the supernatant at a 0.075% (w/v) CHAPS concentration and a CHS concentration 10 fold less than that used in the solubilization step. As shown in Figure 3, the optimal ratio of CHAPS to CHS was about 10:1.

### Comparison of Detergent Concentration for Binding Activity of the Solubilized GRP Receptor

The dependency of binding activity on the concentration of detergent was studied. As is shown in Figure 4, GRP binding to the receptor has a narrow optimum between 0.075% and 0.1% CHAPS, and specific binding falls dramatically at CHAPS concentrations greater than 0.4%. Detergent levels above a concentration of 0.4% also cause a large increase in the nonspecific background in the assay which is possibly due to the formation of detergent aggregates. While the GRP receptor is maximally extracted from membranes with detergent levels that are highly inhibitory (0.75%), the inactivation of receptor molecules by CHAPS appeared to be reversible. Complete binding activity of the receptor incubated in 0.75% CHAPS and 0.15% CHS could be recovered upon reducing the concentration of detergent by dialysis.

### Optimum pH for GRP Binding

$^{125}$I-GRP binding was determined in 500 ml of 20 mM MES, 20 mM CHES, 20 mM HEPES, 2 mM EDTA, 10 mg/ml BSA, 30 μg/ml bacitracin, 0.02 nM $^{125}$I-GRP and 5 μg CHAPS extracted membrane protein at several pH values, ranging from pH 5-10. After incubation at 15°C for 30 minutes, samples were cooled on ice. This was followed by the addition of 5.0 mL of 50 mM HEPES, pH 7.5, to normalize the pH before the separation of bound and free ligand. Receptor binding was found to be optimal at a pH of 7.5. However, the receptor was able to tolerate incubation at a pH of 10 for at least 24 hours at 4°C without loss of activity. In contrast, incubation of the receptor with a pH 5 buffer at 4°C caused a rapid loss of binding activity.

## EXAMPLE 3

### Solubilization of the GRP Receptor for Assays

Swiss 3T3 fibroblast membranes, prepared in Example 1, were suspended at 15 mg protein/ml in 50 mM HEPES, pH 7.5, 1.0 mM EGTA, 100 mM NaCl, 0.25 M sucrose, 50 µg/ml leupeptin, 5 µg/ml pepstatin, 10 µg/ml aprotinin, 30 µg/ml bacitracin, and 0.5 mM phenylmethylsulfonyl fluoride. A mixture of 3-[(3cholamidopropyl) dimethylammonio]-1-propane sulfonate (CHAPS) and cholesteryl hemisuccinate (CHS) in a ratio of 10:1 was added slowly to yield a final concentration of 0.75% CHAPS. The extract was incubated at 21°C for 30 minutes, cooled to 4°C and the insoluble material was removed by centrifugation at 100,000 x gravity for 60 minutes. The clear supernatant was frozen in liquid $N_2$ and stored at -80°c without loss of activity.

## EXAMPLE 4

### Ligand Binding Assays

Specific[125]I-GRP (3-(125Iodotyrosyl15) gastrin releasing peptide, 1900-2000 Ci/mmol) binding to intact or detergent solubilized membranes (20-50 µg, prepared as in Example 3) was assayed in 50 mM HEPES, pH 7.5, 2 mM EDTA, 10 mg/ml bovine serum albumin (BSA), 30 µg/ml bacitracin, and 0.02 nM $^{125}$I-GRP. For assays of detergent solubilized membrane extracts, the final CHAPS detergent concentration was adjusted to between 0.050% and 0.20%. The concentration of CHS was maintained at 1/5 to 1/10 the concentration of CHAPS. Samples were also prepared omitting the BSA. After incubation at 15°C for 30 minutes, samples were cooled to 0°C. Bound ligand ($^{125}$I-GRP:GRP receptor complex) was recovered by rapid filtration through polyethyleneimine treated Whatman GF/B filters, followed by four washes with 4 mls of ice cold Tris buffer (50 mM Tris/Cl, pH 7.5). The filters were counted in an Isodata 500 gamma counter. Nonspecific backgrounds were determined by inclusion of 100 nM unlabeled GRP in the assay to compete for specific binding sites and typically represented 1.5-2% of the specific radioactivity bound. The nonspecific binding could be attributed to a small degree of binding of the $^{125}$I-GRP to the filters. It was found that binding activity of the solubilized receptor is highly dependent on the concentration of the detergent. As shown in Figure 4, GRP binding to the receptor has a narrow optimum between 0.075% CHAPS/0.015% CHS and 0.10% CHAPS/0.02% CHS, and specific binding falls dramatically at CHAPS/CHS concentrations greater than 0.4%/0.08%. Detergent levels above about 0.4% CHAPS with 0.08% CHS present also cause a large increase in the nonspecific background possibly due to the formation of detergent aggregates. Since the receptor is maximally extracted from membranes with detergent levels that are highly inhibitory (0.75% CHAPS), inactivation of the receptor by CHAPS appeared to be reversible. Indeed, complete binding activity of receptor incubated in 0.75% CHAPS plus 0.15% CHS could be recovered upon reducing the concentration of detergent by dialysis.

## EXAMPLE 5

### Receptor Kinetics

Assays were performed for various times of incubation and BSA (10 mg/ml) was either included in the assay or omitted. $^{125}$I-GRP binding to the soluble receptor at 15°C leveled off by 20 minutes and remained constant for up to 2 hours. Omission of the BSA that had been added to prevent proteolysis of the ligand had no significant effect on the binding kinetics.

## EXAMPLE 6

### G Protein Complex

The GRP receptor in Swiss 3T3 fibroblast membranes was found to be G protein coupled. Therefore, the effect of guanylnucleotides on $^{125}$I-GRP binding to soluble receptors was studied. The final detergent concentration was 0.075% CHAPS and 0.015% CHS was present. The G protein coupling of the GRP receptor in intact Swiss 3T3 fibroblast membranes was inferred from the observation that the ligand affinity of the receptor was reduced about ten fold in the presence of the nucleotides GDP and GTP and the non-hydrolyzable GTP analogue GMPPNP. In the presence of $Mg^{+2}$, guanylnucleotides are presumed to induce the dissociation of G proteins from the high affinity ligand/ receptor/G protein ternary complex, resulting in formation of the ligand/receptor complex that displays lower affinity. The GRP receptor extracted from membranes by CHAPS showed no change in their ligand binding properties in the presence of $Mg^{+2}$ and GTP or GMPPNP at levels that reduce GRP binding to membranes by about 80%. The lack of an effect of GTP on GRP binding in the presence of $Mg^{+2}$ indicates that interaction of the receptor with its G protein is not maintained in the detergent extract. The control in the following table, contains $MgCl_2$.

| Guanylnucleotide | Solubilized Membranes Counts/minute Bound Measured as % of Total Added |
|---|---|
| control | 28 |
| control + 10 μM AMPPNP | 27.8 |
| control + 10 μM GTP | 27.5 |
| control + 10 μM GMPPNP | 26.5 |
| control + 10 μM GMPPNP + 100 nM GRP1-27 | 2.0 |
| Guanylnucleotide | Intact Membranes Counts/minute Bound Measured as % of Total Added |
| control | 28.9 |
| control + 5 μM ATP | 29.7 |
| control + 5 μM AMPPNP | 33.4 |
| control + 5 μM GTP | 10.7 |
| control + 5 μM GMPPNP | 10.5 |
| control + 5 μM GMPPNP + 100 nM GRP1-27 | 1.4 |

EXAMPLE 7

Scatchard Analysis of the Soluble GRP Receptors

Scatchard analysis of $^{125}$I-GRP binding to intact and solubilized Swiss 3T3 membranes was done. One particular experiment is discussed below, where the binding parameters of the solubilized and the membrane bound form of the receptor are determined under similar conditions. Assays were determined at 15°C. For assays of solubilized or intact membranes, the binding reactions were terminated at 30 and 180 minutes, respectively. The following are the binding parameters, where KD is the dissociation constant and Bm is the maximum binding capacity:

$$K_D \text{ (intact membranes)} = 37 \text{ pM}$$

$$K_D \text{ (solubilized membranes)} = 10 \text{ pM}$$

$$B_m \text{ (intact membranes)} = 0.79 \text{ pmol/mg protein}$$

$$B_m \text{ (solubilized membranes)} = 1.0 \text{ pmol/mg protein}$$

Scatchard analysis revealed the presence of a high affinity binding site. Some non-linearity and scatter in the data was observed at low values of bound/free ligand where determination of precise binding data is most difficult. The dissociation constant of the ligand binding to the soluble receptors (10 pM) was less than that exhibited by the receptors in intact membranes (37 pM) despite the lack of G protein coupling to the soluble receptors that was observed. As noted above, such G protein coupling boosts the affinity of the membrane receptors by an order of magnitude. However, the assay was performed under conditions that had been optimized for GRP binding to the soluble receptor which may have compensated for the affinity lost by G protein interactions. In other experiments, the dissociation constant of the solubilized receptor was calculated to range from 10 pM to 30 pM. The data demonstrated that the functional conformation of the receptor binding site was maintained in detergent solution.

The Scatchard data from this experiment also indicated that there were 0.79 pmol receptors/mg protein in crude Swiss 3T3 cell membranes and about 50% of the receptor binding sites were solubilized by extracting the membranes with detergent. Some of the factors that were found to be necessary for the most efficient solubilization of receptor activity were inclusion of NaCl (>100 mM), elimination of divalent cations and the extraction of membranes at room temperature. Although NaCl was necessary for the optimal solubilization of the receptors, the salt inhibited GRP binding to both the Swiss 3T3 fibroblast membranes and detergent solubilized receptor ($IC_{50}$= approx. 50 mM). However, the inhibition of the receptors by NaCl at concentrations up to 1.0 M was found to be completely reversible.

EXAMPLE 8

Ligand Specificity of GRP Binding Sites in Soluble Membrane Extracts

The binding of $^{125}$I-GRP to solubilized 3T3 membranes was assayed in the presence of various unlabeled competitor peptides. The C-terminal eight amino acids of GRP (GRP20-27) were found to be essential for high affinity binding to the GRP receptors in whole cells. The complete GRP sequence (GRP127), the N-terminal portion of GRP (GRP1-16), substance P, substance P antagonist, physalemin (all of which were from Peninsula Laboratories, Belmont CA), and the C- terminal portion of GRP with norleucine substituted for methionine referred to as [Nle$^{14,27}$]GRP13-27 ((i.e.Lys-Nle-Tyr-Pro-Arg-Gly-Asn-His-Trp-Ala-Val-Gly-His-Leu-Nle-NH$_2$), were tested for their ability to compete for $^{125}$I-GRP binding to soluble 3T3 fibroblast membrane extracts. The concentration of [Nle$^{14,27}$]GRP13-27 required to cause 50% inhibition of $^{125}$I-GRP binding to the soluble receptor ($IC_{50}$= 0.3 nM) was slightly higher than that of GRP1-27 ($IC_{50}$= 0.1 nM). In contrast, the N-terminal portion (GRP1-16) was unable to compete with $^{125}$I-GRP for binding to the soluble receptor. Additionally, substance P, substance P antagonist and physalemin had no inhibitory effect at the concentrations tested (up to 1000 nM). These results parallel closely that which was found in similar studies in whole cells and isolated membranes.

EXAMPLE 9

Cross-linking of $^{125}$I-GRP Receptors

The molecular weight of the GRP receptor in solubilized Swiss 3T3 membranes was estimated by covalently crosslinking it to bound $^{125}$I-GRP via the homobifunctional crosslinking reagent bis(sulfosuccinimidyl) suberate (BS$^3$) and analyzing the affinity of labeled receptor by SDS-PAGE (sodium dodecyl sulfate-polyacrylamide gel electrophoresis). This cross-linker is specific for primary amino groups. Soluble 3T3 fibroblast membrane protein (40µg) was incubated for 30 minutes at 15°C in a final volume of 500 ml of 50 mM HEPES, 2 mM EDTA, 0.075% CHAPS, 0.015% CHS, 30 µg/ml bacitracin, and 0.2 nM $^{125}$I-GRP. The binding reaction was cooled to 0°C and BS$^3$ was added to yield a final concentration of 3 mM. Cross-linking was quenched by addition of 0.10 ml of Tris buffer (1.0 M Tris/Cl, pH 7.5). After another 10 minute incubation, 0.1 ml TCA (100%) was added and the solution was further incubated at 0°C for 30 minutes. Precipitated material was collected by centrifugation, washed with ice cold acetone, and heated at 95°C for 3 minutes in SDS-PAGE sample buffer. The samples were subjected to SDS-PAGE on a 7.5% gel and the gel was fluorographed. A detailed description of the SDS-PAGE technique is found in Laemmli et al., Nature 227:680 (1970), which is incorporated herein by reference. Figure 5 illustrates the gel display.

| Lane | Composition |
|------|-------------|
| A | no addition |
| B | 0.1 nM unlabeled GRP |
| C | 0.5 nM unlabeled GRP |
| D | 1.0 nM unlabeled GRP |
| E | 100 nM unlabeled GRP |

A strongly labeled species migrated in a diffuse band with an apparent $M_r$ of about 75-100 kDa. Low levels of unlabeled GRP inhibited the labeling of this species, indicating that the labeling is highly specific. The broadness of the labeled band is consistent with the fact that the GRP receptor has been found to contain carbohydrate. The labeled product is very similar to that derived from whole cell or membrane crosslinking experiment. N-Glycanase treatment of samples derived from cross-linked whole cells indicated that the labeled protein contained N-linked carbohydrates. The deglycosylated protein displayed an apparent $M_r$ of 38 kDa on SDS-PAGE.

EXAMPLE 10

Purification of the GRP Receptor Solubilization of the GRP Receptor

Swiss 3T3 fibroblast membranes (2-3 g of protein) were prepared as described in Example 1 and suspended in 200 ml storage buffer (see Example 1). The membranes were mixed with 50 ml of NaC1 (5.0 M), bringing the NaCl concentration to about 1 M, pelleted by centrifugation at 40,000 x g for 30 minutes, and washed twice at 4°C with 200 ml of high salt buffer (50 mM HEPES, pH 7.5, 2 mM EDTA, 1.0 M NaCl, 25 µg/ml leupeptin, 10 µg/ml aprotinin, 2.5 µg/ml pepstatin and 0.5 mM PMSF). The membranes were then washed with low salt buffer (50 mM HEPES, pH 7.5, 2 mM EDTA, 25 µg/ml leupeptin, 10 µg/ml aprotinin, 2.5 µg/ml pepstatin and 0.5 mM PMSF) and resuspended in 200 ml 50 mM HEPES, pH 7.5, 2 mM EDTA, 1 mM EGTA, 100 mM NaCl, 0.03 mg/ml bacitracin, 25 µg/ml leupeptin, 10 µg/ml aprotinin, 2.5 µg/ml pepstatin and 0.5 mM PMSF. A stock solution containing a mixture of CHAPS and CHS was added slowly to the membranes to give a final concentration of 0.75% CHAPS and 0.075% CHS. The mixture was incubated for 30 minutes at 21°C, cooled to 4°C and centrifuged at 100,000 x g for 60 minutes at 4:C. The supernatant contained the solubilized GRP receptor.

Pecipitation by Polyethylene Glycol

To the solubilized extract (190 ml), 126 ml of ice cold polyethylene glycol (PEG) 8,000 (50 w/v% in $H_2O$) was added. After thorough mixing, the precipitate that formed was collected by centrifugation at 100,000 x g for 10 minutes. The pellet was suspended in 25 mM HEPES, 25 mM Tris, pH 7.5, 2 mM EDTA, 0.075% CHAPS, 0.0075% CHS, 5 µg/ml leupeptin and 10 µg/ml bacitracin in a total volume of 50 ml with the aid of a Potter-Elvehjem homogenizer. The suspension, which contained some insoluble protein, was centrifuged at 69,000 x g for 10 minutes, and the pellet was discarded.

Wheat Germ Agglutinin Chromatography

Following precipitation by PEG, the GRP receptor was further purified by lectin affinity chromatography. A column (1.6 x 9 cm) containing wheat germ agglutinin-agarose resin (3-5 mg lectin/mg of wet gel) (E-Y Laboratories, San Mateo, CA) was equilibrated with 50 mM HEPES, pH 7.5, 2 mM EDTA, 0.25% CHAPS, 0.025% CHS, 5 µg leupeptin and 10 µg bacitracin at 4°C. The soluble extract was diluted with one volume of column buffer, and the final detergent concentration was adjusted to 0.25% CHAPS and 0.025% CHS. The sample was applied to the lectin column at a flow rate of 1.5 ml/min. The column was then washed with about 10 column volumes of buffer, and eluted with column buffer plus 5 mM N,N',N"-triacetylchitotriose. Fractions containing the GRP receptor binding activity were pooled and diluted with 2.3 volumes of 25 mM HEPES, 25 mM Tris, pH 7.5, 2.0 mM EDTA, 5 µg/ml leupeptin and 10 µg/ml bacitracin.

GRP Affinity Chromatography

Actigel superflow resin (10 ml) (Sterogene, San Gabriel, CA) was washed with 5 volumes of 100 mM KPO4, pH 7.0. The resin was incubated with 10 ml of 100 mM KPO4, 100 mM NaCNBH3, pH 7.0 containing 2 mg/ml [Nle[14,27]] GRP13-27 for 2 hours with gentle agitation. The resin was washed with 100 mM KPO4, pH 7.0, followed by alternating washes with 100 mM KAc, pH 4.0, 0.5 M NaCl and 100 mM Tris pH 8.0, 0.5 M NaCl. A column of the resin (1.6 x 5 cm) was equilibrated with 25 mM Tris, 25 mM HEPES, pH 7.5, 2.0 mM EDTA, 0.075% CHAPS, 0.0075% CHS, 5 µg/ml leupeptin and 10 µg/ml bacitracin at 4°C. The crude GRP receptor eluted from the lectin column was loaded onto the GRP affinity column at 0.1 ml/min. The column was then washed with about 20 volumes of the equilibration buffer. The bound receptor was eluted from the column with equilibration buffer plus 0.5 M NaCl at a flow rate of 0.2 ml/min. Fractions containing the receptor were identified by assays of $^{125}$I-GRP binding activity and were pooled (10-13 ml). The elution pool was concentrated to about 1 ml by ultrafiltration using a Centriprep-10 device (Amicon, Danvers, MA). The sample was then desalted by dilution of the sample with 15 volumes of affinity column equilibration buffer and re-concentration of the sample to 1 ml. This desalting step was repeated and the resulting 1 ml sample was diluted to 5 ml with affinity column equilibration buffer. PAGE analysis of the purified GRP receptor revealed the presence of a significant level of contamination.

This solution of semi-pure receptor was loaded onto a second [Nle[14,27]]GRP13-27-actigel superflow column (1.0 x 3 cm), prepared as described above, at 1.8 ml/h. The column was washed with 20 column volumes of equilibration buffer, and the bound receptor was eluted with equilibration buffer plus 0.5 M NaCl at a flow rate of 0.1 ml/min. Fractions containing GRP receptor binding activity were pooled and concentrated to 0.3 ml by ultrafiltration.

Gel Filtration

The purified receptor was desalted by chromatography on a Superose-6 HR 10/30 column (Pharmacia LKB, Piscataway, NJ). The column was equilibrated with 20 mM HEPES, pH 7.5, 2 mM EDTA, 0.075% CHAPS, 0.0075% CHS and 100 mM NaCl. The receptor was chromatographed at 0.4 ml/min. The receptor was eluted from the column in about 2 ml.

Characterization of the Purified GRP Receptor

The overall yield of the pure GRP receptor from the crude solubilized extract ranged from 10-20%, based on recovery of high affinity $^{125}$I-GRP binding activity. Scatchard analysis of binding data obtained with the purified receptor indicated that its affinity for GRP ($K_d$ = 10-30 pM) was essentially the same as the receptor in the crude detergent solubilized extract. The data show that 30-50 pmoles of receptor sites are typically obtained in the final purified fractions of the receptor, as outlined in this example. This corresponds to about 1-2 µg of receptor protein, taking into account that the deglycosylated receptor exhibits an apparent molecular weight of 36%5 kilodaltons on SDS PAGE gels.

A silver stained SDS-PAGE gel of the receptor preparation showed a single intensely staining diffuse band with an apparent molecular weight of 70-100 kD. The receptor preparation was essentially free of contaminants. Figure 6 illustrates the silver stained gel display of the purified GRP receptor. The relative level of silver staining of the GRP receptor band was compared with known amounts of protein to determine the approximate amount of receptor protein loaded on the gel. The rough value obtained was in the range of that estimated to be present by Scatchard analysis of $^{125}$I-GRP binding data, which confirmed that the intensely staining band on the gel was the GRP receptor. Furthermore, the apparent molecular weight of the purified GRP receptor corresponded to that obtained with affinity labeled receptor. This was obtained by binding $^{125}$I-GRP to the receptor in whole cells, intact membranes, or crude soluble extracts, and cross-linking the receptor-ligand complex with a homobifunctional cross-linking reagent.

The diffuse nature of the GRP receptor band on SDS PAGE is characteristic of proteins containing carbohydrate. A small portion of the purified receptor was radiolabeled by iodination with $^{125}$I-NaI in the presence of Iodogen (Pierce, Rockford, IL) to enhance the detection of the receptor on gels. Treatment of the radiolabeled receptor with N-glycanase resulted in loss of the 70-100 kD band, and the generation of a new band at about 36±5 kilodaltons, representing the deglycosylated receptor.

Determination of Partial Amino Acid Sequence of the GRP Receptor

A partial sequence near the N-terminus of the purified GRP receptor was determined by sequential Edman degradation. The sequence obtained for residues 8-17 was:

**-Leu-Asn-Leu-Asp-Val-Asp-Pro-Phe-Leu-Ser-**

EXAMPLE 11

Trypsinization of the Purified GRP Receptor and the Isolation of Tryptic fragments

Purified GRP receptor was prepared as described in Example 10. After Superose-6 chromatography, 40 picomoles of receptor were obtained based on Scatchard analysis of $^{125}$I-GRP binding data. This corresponded to about 1.6 µg of protein. The sample (3 ml) was concentrated to about 100 µl by ultrafiltration using a Centricon-l0 device (Amicon). The sample was then diluted with 2 ml of $H_2O$, and concentrated to 100 µl. Once again, the sample was diluted with 2 ml $H_2O$, and concentrated to 100 µl, and was finally diluted with 1 ml of $H_2O$, and concentrated to 138 µl. To digest the receptor with trypsin, 0.1 µg of trypsin was added, and the sample was incubated at 37°. After 2 hours, an additional 0.1 µg of trypsin was added, followed by another 0.2 µg of trypsin after 5 hours of incubation. After 22 hours at 37°C, the sample was rapidly frozen in liquid $N_2$ and stored at -80°C.

Trypsin digested GRP receptor was thawed to room temperature and reduced with dithiothreitol (DTT) at a final concentration of 10 mM for 30 minutes at 37°C. The entire DTT treated tryptic digest was then fractionated by reverse phase high pressure liquid chromatography (HPLC) using a 2.1 mm X 3 cm C4 column (Brownlee, Aquapore Butyl, 300 angstrom pore size), and a linear gradient of 0.05% trifluoroacetic acid (TFA) in water (solvent A) to 0.05% TFA in 100% acetonitrile (solvent B) (Figure 7). The conditions for the HPLC gradient were 0% solvent B to 100% solvent B in 60 minutes at a flow rate of 0.2 milliliters per minute. Effluent fractions were detected at 215 nm, collected at one minute intervals, and stored at 4°C.

For peptide sequence analysis, consecutive fractions were pooled and concentrated on a Speed Vac (Savant, Farmingdale, NY) to a final volume of approximately 50 µl. The sample was loaded in entirety onto a glass fiber filter which had been treated and precycled with Biobrene (Applied Biosystems (ABI), Foster City, CA). Automated amino acid sequence analysis was performed on an ABI
model 475A gas phase sequencer (Hewick et al., J.Biol.Chem. 256:7990-7997, 1981) equipped with an ABI model 120A on-line detection HPLC system for identification of phenylthiohydantoin (PTH-) amino acids. Quantitation of PTH-amino acids was performed by an ABI model 900 data system using 60 picomoles of a set of known PTH-amino acid standards (ABI). In this manner, the combined tryptic HPLC fractions 56 through 59 gave the amino acid sequence MASFLVFYVIPLAII (designated T56/59); the tryptic HPLC fraction 44 yielded the amino acid sequence QLTSVGVSV (designated T44), and the tryptic HPLC fraction 50 gave the amino acid sequence PNLFISXLALG (designated T50), where X denotes a residue that could not be identified.

NH$_2$-terminal sequence analysis was performed on the intact purified GRP receptor following washing of the sample with H$_2$O and concentration of the sample on a Centricon 10 ultrafiltration device (Amicon, Danvers, MA). The sample (95% or approximately 95 µl was loaded onto a Biobrene (ABI) precycled glass filter and NH$_2$-terminal sequence analysis was performed through 30 cycles of automated Edman degradation on an ABI 475A gas phase sequencer (Hewick et al.). PTH- amino acid identification and quantitation were performed using an ABI 120A PTH-amino acid analyzer and ABI 900 data system. Following two separate NH$_2$-terminal sequence runs on two purified preparations of the GRP receptor, the following consensus NH$_2$-terminal amino acid sequence was obtained for 17 residues, where X denotes a residue for which an accurate assignment of a specific amino acid was not made:

```
      1         5         10        15
      A  P  N  X  X  S  X  L  N  L  D  V  D  P  F  L  S
```

EXAMPLE 12

Identification of cDNA Clone

Encoding the Swiss 3T3 GRP Receptor

Preliminary studies established that a murine embryonal fibroblast cell line (Balb 3T3) expressed a repertoire of mRNAs very similar in abundance and distribution to the GRP receptor- expressing Swiss 3T3 murine fibroblast cell line, but did not have any cell surface GRP receptors detectable in standard binding assays (Kris et al., J.Biol.Chem. 262:11215-11220, 1987; Zachary et al., Proc.Natl.Acad.Sci. USA 82:7616-7620, 1985). These observations suggested that the GRP receptor mRNA would be one of a limited number of transcripts present in Swiss 3T3, but absent from Balb 3T3 mRNA. Polyadenylated mRNA was isolated from both Swiss 3T3 and Balb 3T3 cell lines and was used to generate a Swiss 3T3 subtracted cDNA library enriched for cDNAs derived from Swiss 3T3 mRNA but not represented in Balb 3T3 mRNA using published methodology (Timlin et al., Nuc.Acids.Res. 18:1587-1593, 1990, incorporated herein by reference). The cDNA inserts whose length exceeded 300 base pairs were ligated into the lambda gtlO bacteriophage cDNA cloning vector and the library amplified using established methods (Davis et al., Basic Methods in Molecular Biology, Elsevier Science Publishing Company, New York, 1986).

The library was screened with an oligonucleotide probe whose sequence was based on the amino acid sequence of an internal tryptic fragment (T 56/59) purified by HPLC from a tryptic digest of the purified GRP receptor protein. The amino acid sequence (MASFLVFYVIPLAII) of the internal peptide was used to design a long non-degenerate antisense oligonucleotide whose sequence was based on optimal codon usage frequency as described in the literature (Lathe, Mol.Biol. 183:1-12, 1985), resulting in a 44-base long probe referred to as I3: (5'ATGATGGCCAGGGGGATCACATAGAAGACCAGGAAGGAGGCCAT 3'). The I3 probe was labelled by phosphorylation of the 5' end using gamma 32P- ATP and polynucleotide kinase employing established techniques (Davis et al.). The labelled probe was used to screen 100,000 member clones from the subtracted library using hybridization and wash conditions previously described (Wood, Chapter 48 in Methods in Enzymology 152:443-447, 1987). Duplicate screening identified five positive clones, which were plaque purified. The EcoRI inserts from the five clones were subcloned into the plasmid vector pGEM 4 (Promega), and the nucleotide sequence of the hybridizing inserts was determined using the Sequenase 2.0 double stranded sequencing kit (US Biochemical). Two of the five clones (T1 and T2) had an identical region of overlapping DNA sequence which encoded the internal peptide used to design the oligonucleotide probe. The fragment was removed from the plasmid vector by EcoRI digestion and purified by gel electrophoresis and electroelution as described (Davis et al.). The purified insert fragments were labelled by random primer extension using a commercially available kit and the supplier's recommendations (Bethesda Research Laboratories) to generate a probe to identify other overlapping cDNA clones from the subtracted library in a second screening of the 100,000 library members. Nucleotide sequence analysis of the nine additional clones identified revealed a single long open reading frame whose predicted translation product included the internal tryptic fragment amino acid sequence, which ended in a termination codon within the composite sequence. The amino terminal end of the open reading frame was not present in any of the clones isolated from the subtracted library.

To obtain the 5' end of the cDNA and at the amino terminal end of the open reading frame, an in vitro polymerase chain reaction amplification (PCR) cDNA cloning procedure (5' RACE) was performed essentially as described in Frohman et al., Proc.Natl.Acad.Sci. USA 85:8998-9002, 1988) using two nested gene-specific oligonucleotides (EXT 3:

5' GGGGAGCCAGCAGAAGGC 3'; EXT 2: 5' CCATGGAATGGATTTTA) derived from the known nucleotide sequence of the cDNA clones previously analyzed. EXT 3 was used as a gene-specific primer for reverse transcription of Swiss 3T3 mRNA, and EXT 4 was used as a gene specific primer for Taq DNA polymerase catalyzed PCR. Nineteen 5' RACE cDNAs were isolated and characterized, and five of the clones that extended the longest distance were sequenced as described previously. Nucleotide sequence analysis revealed an extension of the long open reading frame encoding the internal tryptic peptide amino acid sequence, beginning with an initiator methionine codon. The predicted amino acid sequence of the open reading frame was compared with amino terminal sequence derived from the purified GRP receptor (Example 11). The experimentally determined amino acid sequence did not contain the methionine at position 1 of the deduced sequence, but corresponded well to residues 2-18. Deduced amino acids 2-4 and 8-18 (Figure 8A) were identical. The amino acids that did not match (amino acids 5-7, Figure 8A) were ambiguous in the original amino acid sequence, probably because they are located at an N-linked glycosylation site (Asn-Cys-Ser). In addition, the amino acid sequence from internal tryptic peptides T44 (QLTSVGVSV) and T50 (PNLFISXLALG), derived from the purified Swiss 3T3 GRP receptor (Example 11), matched segments within the long open reading frame of the composite GRP receptor cDNA.

Gene-specific primer-directed cDNA cloning was used to obtain a single cDNA clone which encodes the entire uninterrupted open reading frame. In this procedure, a genespecific oligonucleotide (EXT 7: 5' TACTTTGAGATACAATGG 3') complementary to an 18 nucleotide segment of the 3' untranslated region of the GRP receptor mRNA was used to prime the synthesis of first-strand cDNA by MuLV reverse transcriptase. Double-stranded cDNA was generated, and cloned into lambda gtlO using standard methodology (Davis et al.). Five hundred thousand clones were screened with a cDNA fragment probe derived from one of the 5' RACE cDNA clones which extended into the 5' untranslated region of the cDNA. Over twenty clones were identified, and ten were plaque purified and subcloned into plasmid vectors by standard methods (Davis et al.). Nucleotide sequence analysis confirmed that the clones contained the entire uninterrupted open reading frame of the GRP receptor protein. The DNA sequence of the GRP receptor and its deduced amino acid sequence is shown in Figure 8.

Analysis of the nucleotide sequence of the open reading frame revealed several interesting features of the predicted protein. The predicted molecular weight of the protein is about 43,100 daltons, in good agreement with that reported for the N-glycanase reduced GRP binding protein from Swiss 3T3 cells, described in Example 10. Hydrophobicity analysis predicts the presence of seven putative transmembrane domains (Figure 9), consistent with earlier observations that the GRP receptor is coupled to a guanine-nucleotide binding protein (G-protein) (Fischer et al., J. Biol. Chem. 263:28082816, 1988). The superfamily of G-protein coupled receptor genes typically share certain conserved residues within or adjacent to the seven transmembrane domains (Masu et al., Nature 329:836-838, 1987). These conserved amino acids are found in the predicted locations within the open reading frame of the GRP receptor sequence (Figure 8). Five potential sites for N-linked glycosylation (Asn-X-Ser/Thr) are noted (Figure 8), consistent with the observation that the GRP receptor is heavily glycosylated, and that N-glycanase treatment of the GRP receptor glycoprotein reduces the apparent molecular weight of the protein in SDS polyacrylamide gels from about 70-100 kilodaltons to about 38±5 kilodaltons (Example 10).

Northern blot analysis (Davis et al.) was undertaken to identify the nature of the transcripts encoding the Swiss 3T3 GRP receptor (Figure 10). One microgram of polyadenylated mRNA derived from Swiss 3T3 and Balb 3T3 cells was purified and resolved by electrophoresis on a formaldehyde-containing one percent agarose gel, which was subsequently transferred to a nitrocellulose filter. The filter was hybridized with a 450base pair cDNA fragment probe encoding the carboxy terminal transmembrane domains 5, 6, and 7 as well as a portion of the 3' untranslated sequences. The probe was labelled with 32P to a specific activity 500 cpm/picogram using a commercially available random primer extension kit (Bethesda Research Laboratories). Two mRNAs specifically hybridized to the probe, whose sizes were estimated to be 7.2 kb and 3.0 kb by comparison to mouse 28S (5.0 kb) and 18S (2.0 kb) markers (Figure 10). As expected, the two mRNA forms were only detected in mRNA from Swiss 3T3, with no GRP receptor transcripts observed in mRNA from Balb 3T3 cells.

EXAMPLE 13

Human mRNA Species Homologous to Mouse GRP Receptor cDNA

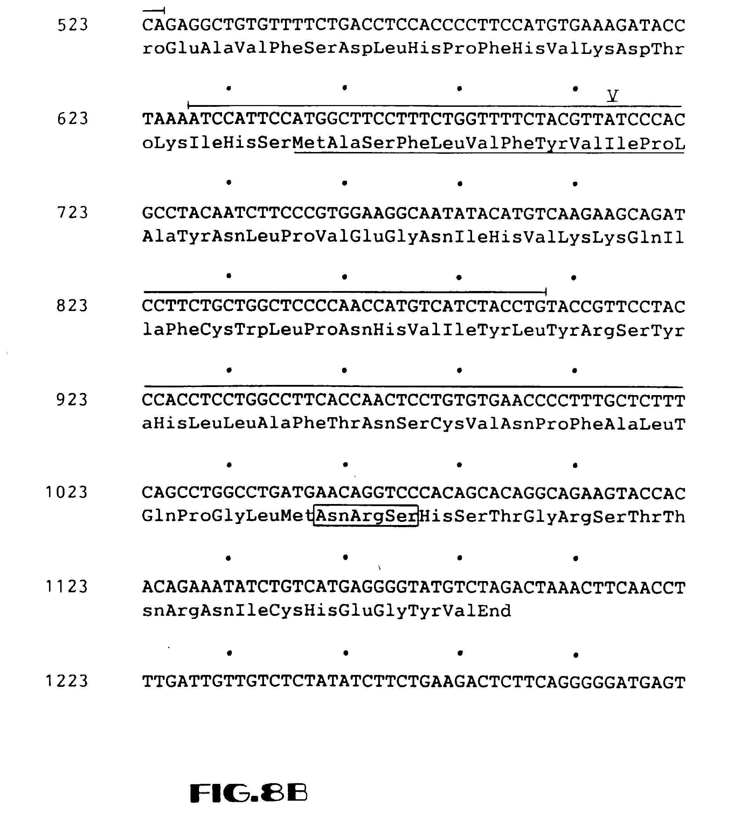

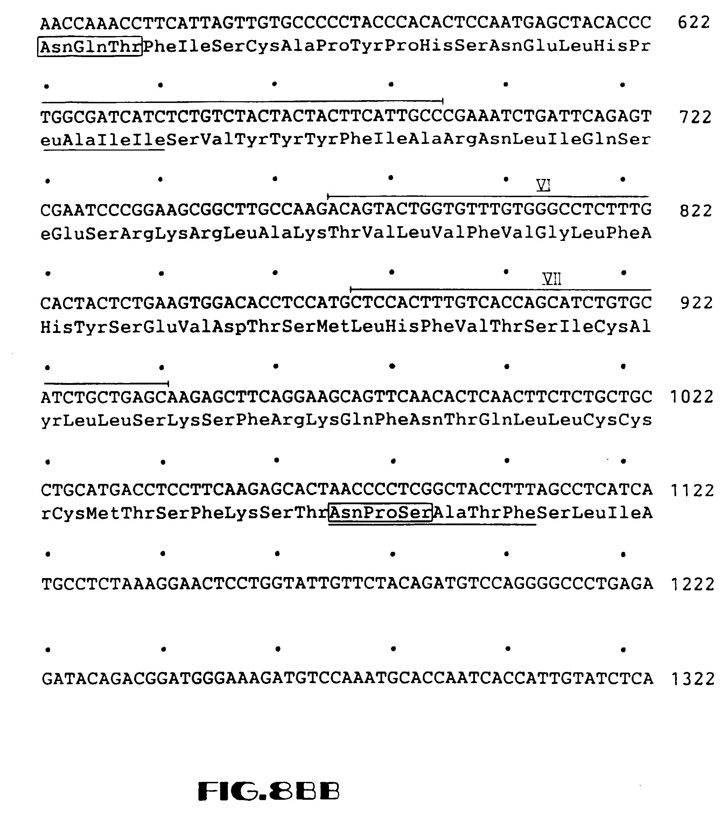

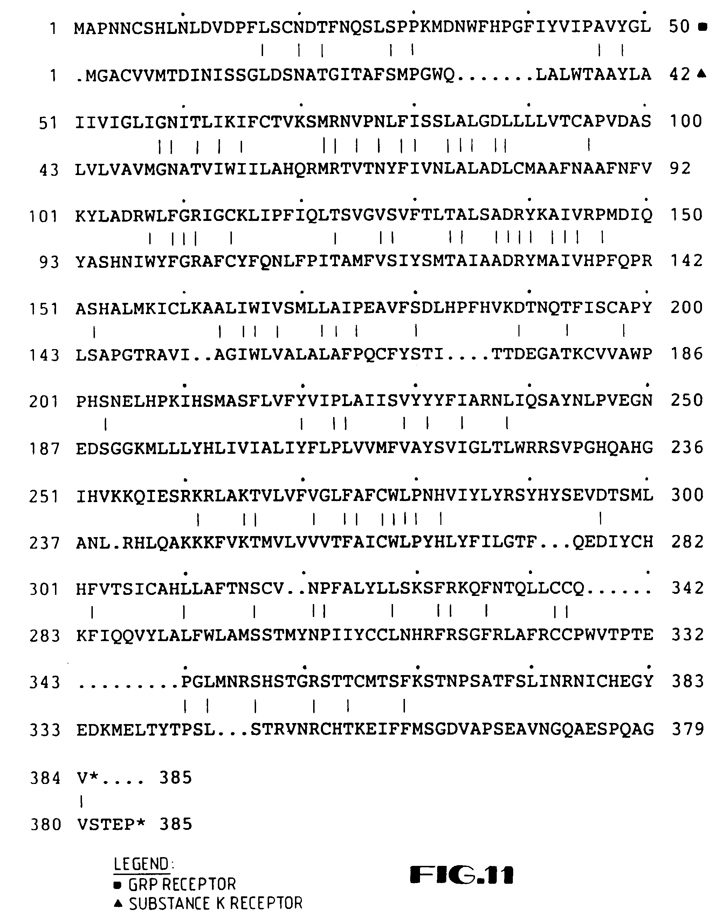

Northern blot analysis was performed to determine the degree of homology between the GRP receptor expressed in human fetal lung cells (Kris et al., J.Biol.Chem. 262:1121511220, 1987) and the Swiss 3T3 cell receptor. Polyadenylated mRNA was isolated from human fetal lung cells, and subjected to Northern analysis as described in Example 12, using the same 450-base pair cDNA fragment of the Swiss 3T3 cell GRP receptor as a probe, except that the stringency of the hybridization filter washing steps was reduced. Two mRNA species of approximately 7.2 and 3.0 kb were detected in the human cell line, corresponding to those observed in mouse Swiss 3T3 cell mRNA (Figure 12). Based on the conditions used for the blot, the mRNA species identified were at least 80% homologous to the Swiss 3T3 GRP receptor probe. The results indicate that the mouse GRP receptor cDNA, described in Example 12, can be used to readily isolate cDNAs or genomic DNA fragments encoding the GRP receptor in other mammalian species, including humans.

EXAMPLE 14

Expression of the Mouse GRP Receptor Derived from the cDNA Clone in Xenopus Oocytes to Demonstrate Receptor Function

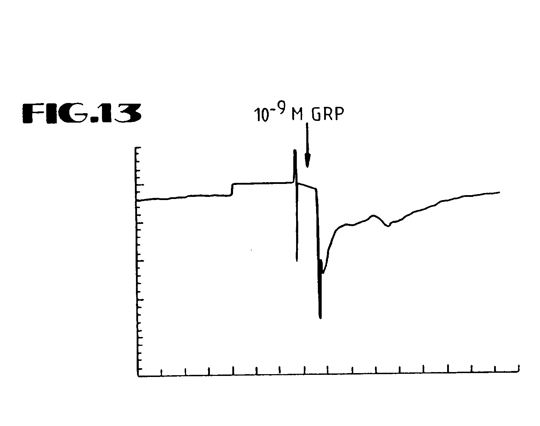

A sense in vitro transcript was prepared from the mouse GRP receptor cDNA protein coding region (Figure 8) cloned in the transcription vector pGEM 4 (Promega) using sp6 RNA polymerase and established methods (Davis et al., 1986). The synthesized transcript (about 20 nanograms) was injected into Xenopus oocytes. Sixteen hours later, the oocytes were voltage clamped and bathed in a solution containing $10^{-9}$ M GRP. As shown in Figure 13, a GRP ligand dependent chloride current (magnitude of about 160 nanoamperes), was coincident with addition of the ligand. These results demonstrate the expression of an in vitro transcript dependent GRP receptor on the Xenopus oocyte cell surface, which is coupled through Gproteins to a $Ca^{++}$ dependent chloride channel. The ligand dependent chloride current was not observed in control oocytes injected with an antisense in vitro transcript demonstrating specificity of the response.

**Claims**

1. A method for solubilizing the gastrin releasing peptide receptor in active form comprising the steps of:

   (1) preparing membranes from a gastrin releasing peptide receptor source;

   (2) washing said membranes and suspending said membranes in a buffer;

   (3) solubilizing the gastrin releasing peptide receptor by incubating said membranes in a solution to which a detergent and a soluble cholesteryl ester has been added; and

   (4) adjusting the detergent concentration of said solubilized gastrin releasing peptide receptor.

2. The method of claim 1, wherein said gastrin releasing peptide receptor source is a eukaryotic or prokaryotic host expressing recombinant gastrin releasing peptide receptor.

3. A method for solubilizing and purifying the gastrin releasing peptide receptor in an active form comprising the steps of:

   (1) preparing membranes from a gastrin releasing peptide receptor source;

   (2) washing said membranes and suspending said membranes in a buffer;

   (3) solubilizing the gastrin releasing peptide receptor by incubating said membranes in a solution to which a detergent and a soluble cholesteryl ester has been added, forming a soluble extract;

   (4) precipitating the gastrin releasing peptide receptor from the soluble extract of step (3), collecting the precipitate by centrifugation and resuspending said precipitate in a buffer containing said detergent and soluble cholesteryl ester, forming a soluble extract;

   (5) applying the soluble extract of step (4) to a wheat germ agglutinin affinity column, forming an eluate;

   (6) further fractionating the wheat germ agglutinin column eluate from step (5) on a gastrin releasing peptide affinity column forming an eluate; and

   (7) dialyzing the gastrin releasing peptide affinity column eluate from step (6) and applying said eluate to a second gastrin releasing peptide affinity column.

4. The method of claim 3 which further comprises a gel filtration step following step (7).

5. The method of claim 3 wherein said gastrin releasing peptide receptor source is a mammalian cell line.

6. Solubilized gastrin releasing peptide receptor in an active form.

7. The receptor of claim 6 which is solubilized by:

> (1) preparing membranes from a gastrin releasing peptide receptor source;

> (2) washing said membranes and suspending said membranes in a buffer;

> (3) solubilizing the gastrin releasing peptide receptor by incubating said membranes in a solution to which a detergent and a soluble cholesteryl ester has been added; and

> (4) adjusting the detergent concentration of said gastrin releasing peptide receptor.

8. The receptor of claim 7 wherein said gastrin releasing peptide receptor source is a eukaryotic or prokaryotic host expressing recombinant gastrin releasing peptide receptor.

9. The receptor of claim 7 wherein said gastrin releasing peptide receptor source is a mammalian cell line.

10. The receptor of claim 7 wherein said detergent is 3-[(3-cholamidopropyl)dimethylammonio]-1-propane sulfonate and said soluble cholesteryl ester is cholesteryl hemisuccinate.

11. The receptor of claim 6 which binds gastrin releasing peptide with an affinity of at least $K_D$=10 nM.

12. The receptor of claim 6 having the following characteristics:

> runs as a broad band on SDS-PAGE with an apparent molecular weight of about 70 to 100 kilodaltons;

> binds selectively with polypeptides of the bombesin type;

> has a $K_D$ value of about 10-100 pM;

> is free of coupled G proteins;

> contains N-linked carbohydrates;

> when deglycosylated, has an apparent molecular weight of 36±5 kilodaltons on SDS-PAGE; and

> has a partial amino acid sequence near the N-terminus of:

**-Leu-Asn-Leu-Asp-Val-Asp-Pro-Phe-Leu-Ser-.**

13. Solubilized and purified gastrin releasing peptide receptor in an active form which is substantially free of contaminating impurities.

14. The receptor of claim 13 which is solubilized and purified by:

> (1) preparing membranes from a gastrin releasing peptide receptor source;

> (2) washing said membranes and suspending said membranes in a buffer;

> (3) solubilizing the gastrin releasing peptide receptor by incubating said membranes in a solution to which a detergent and a soluble cholesteryl ester has been added, forming a soluble extract;

(4) precipitating the gastrin releasing peptide receptor from the soluble extract of step (3), collecting the precipitate by centrifugation and resuspending said precipitate in a buffer containing said detergent and soluble cholesteryl ester, forming a soluble extract;

(5) applying the soluble extract of step (4) to a wheat germ agglutinin affinity column, forming an eluate;

(6) further fractionating the wheat germ agglutinin column eluate from step (5) on a gastrin releasing peptide affinity column, forming an eluate; and

(7) dialyzing the gastrin releasing peptide affinity column eluate from step (6) and applying said eluate to a second gastrin releasing peptide affinity column.

15. The receptor of claim 14 which is solubilized and purified by the further step of gel filtration following step (7).

16. The receptor of claim 13 having the following characteristics:

runs as a broad band on SDS-PAGE with an apparent molecular weight of about 70 to 100 kilodaltons;

binds selectively with polypeptides of the bombesin type;

has a $K_D$ value of about 10-100 pM;

is free of coupled G proteins;

contains N-linked carbohydrates;

when deglycosylated, has an apparent molecular weight of $36\pm5$ kilodaltons on SDS-PAGE; and

has a partial amino acid sequence near the N-terminus of:

**-Leu-Asn-Leu-Asp-Val-Asp-Pro-Phe-Leu-Ser-** .

17. Antibodies having binding affinity to naturally occurring gastrin releasing peptide receptor.

18. The antibodies of claim 17 which are raised against the GRP receptor, or fragments thereof.

19. The antibodies of claim 17 which are conjugated to radionuclides.

20. A method of screening for compounds having binding affinity to the gastrin releasing peptide receptor which comprises utilizing a source of gastrin releasing peptide receptor, which is soluble and active and substantially free of other gastrin releasing peptide binding protein, in a receptor/labeled compound binding assay comprising the steps of:

(1) contacting and incubating said source of gastrin releasing peptide receptor with a labeled compound having known binding affinity to the gastrin releasing peptide receptor and a test compound whose binding affinity to the gastrin releasing peptide receptor is being evaluated;
(2) separating the bound from free labeled compound; and
(3) measuring the degree of labeled compound binding, said value being inversely proportional to the amount of test compound binding.

21. The method of claim 20 wherein said source of gastrin releasing peptide receptor is a eukaryotic or prokaryotic host cell which has been stably transformed with recombinant DNA molecules expressing the gastrin releasing peptide receptor.

22. The method of claim 20 wherein said source of gastrin releasing peptide receptor is membranes from a eukaryotic or prokaryotic cell which has been stably transformed with DNA vectors directing the expression of the gastrin releasing peptide receptor.

**23.** The method of claim 20 wherein said solubilized receptor is purified.

**24.** The method of claim 20 wherein said labeled compound is a ligand or an antibody.

**25.** A kit for determining the binding affinity of a test compound to the gastrin releasing peptide receptor comprising a labeled compound having known binding affinity for the gastrin releasing peptide receptor, gastrin releasing peptide receptor in an active form and which is substantially free of other gastrin releasing peptide binding protein and a means for separating bound from free labeled compound.

**26.** The kit of claim 25 wherein said means for separating is a solid phase for immobilizing the gastrin releasing peptide receptor.

**27.** The kit of claim 25 wherein said labeled compound is a ligand or an antibody.

**28.** A method for the determination of the concentration of gastrin releasing peptide receptor in a sample comprising the steps of:

(1) preparing membranes from a sample comprised of a gastrin releasing peptide receptor source;

(2) washing said membranes and suspending said membrane in a buffer;

(3) solubilizing the gastrin releasing peptide receptor by incubating said membranes in a solution to which a detergent and a soluble cholesteryl ester has been added;

(4) adjusting the detergent concentration of said solubilized gastrin releasing peptide receptor;

(5) contacting and incubating said dilution with radio labeled gastrin releasing peptide to form gastrin releasing peptide: gastrin releasing peptide receptor complexes;

(6) recovering said complexes; and

(7) measuring the radioactivity of said recovered complexes.

**29.** A kit for determining the concentration of gastrin releasing peptide receptor in a sample comprising a labeled compound having known binding affinity for the gastrin releasing peptide receptor, gastrin releasing peptide receptor in an active form and which is substantially free of other gastrin releasing peptide binding protein and a means for separating bound from free labeled compound.

**30.** The kit of claim 29 wherein said means for separating is a solid phase for immobilizing the gastrin releasing peptide receptor.

**31.** The kit of claim 29 wherein said labeled compound is a ligand or an antibody.

**32.** Antibodies having binding affinity to naturally occurring gastrin releasing peptide receptor for use in the treatment of a disease or disorder associated with abnormal expression or abnormal triggering of the gastrin releasing peptide receptor.

**33.** Antibodies of claim 32, wherein said gastrin releasing peptide receptor is solubilized in an active form and said antibodies recognize epitopes which are only present in the active gastrin releasing peptide receptor.

**Patentansprüche**

**1.** Verfahren zur Solubilisierung des Rezeptors für das Gastrin-freisetzende Peptid in aktiver Form, folgende Schritte umfassend:

(1) Herstellen von Membranen aus einem Ausgangsmaterial eines Rezeptors für das Gastrin-freisetzende Peptid;

(2) Waschen dieser Membranen und Suspendieren dieser Membranen in einer Pufferlösung;

(3) Solubilisieren des Rezeptors für das Gastrin-freisetzende Peptid durch Inkubieren der Membranen in einer Lösung, der ein Detergens und ein löslicher Cholesterylester zugegeben wurde; und

(4) Einstellen der Detergens-Konzentration des solubilisierten Rezeptors für das Gastrin-freisetzende Peptid.

2. Verfahren nach Anspruch 1, wobei das Ausgangsmaterial des Rezeptors für das Gastrin-freisetzende Peptid ein eukaryotischer oder prokaryotischer Wirt ist, der einen rekombinierten, Gastrin-freisetzenden Peptid-Rezeptor exprimiert.

3. Verfahren zur Solubilisierung und Reinigung des Rezeptors für das Gastrin-freisetzende Peptid in aktiver Form, folgende Schritte umfassend:

(1) Herstellen von Membranen aus einem Ausgangsmaterial eines Rezeptors für das Gastrin-freisetzende Peptid;

(2) Waschen der Membranen und Suspendieren der Membranen in einer Pufferlösung;

(3) Solubilisieren des Rezeptors für das Gastrin-freisetzende Peptid durch Inkubieren der Membranen in einer Lösung, der ein Detergens und ein löslicher Cholesterylester zugegeben wurde, um einen löslichen Extrakt zu bilden;

(4) Fällen des Rezeptors für das Gastrin-freisetzende Peptid aus dem löslichen Extrakt aus Verfahrensschritt (3), Gewinnung des Niederschlags durch Zentrifugierung und Resuspendierung des Niederschlags in einer Pufferlösung, die das Detergens und den löslichen Cholesterylester enthält, um einen löslichen Extrakt zu bilden;

(5) Aufbringen des löslichen Extrakts aus Verfahrensschritt (4) auf eine Weizenkeim-Agglutinin-Affinitätssäule unter Bildung eines Eluats;

(6) weiteres Fraktionieren des Eluats der Weizenkeim-Agglutinin-Säule aus Verfahrensschritt (5) auf einer Affinitätssäule für das Gastrin-freisetzende Peptid unter Bildung eines Eluats; und

(7) Dialysieren des Eluats der Affinitätssäule für das Gastrin-freisetzende Peptid aus Verfahrensschritt (6) und Aufbringen dieses Eluats auf eine zweite Affinitätssäule für Gastrin-freisetzendes Peptid.

4. Verfahren nach Anspruch 3, das weiterhin den Verfahrensschritt einer Gelfiltration im Anschluß an Verfahrensschritt (7) umfaßt.

5. Verfahren nach Anspruch 3, wobei das Ausgangsmaterial des Rezeptors für das Gastrin-freisetzende Peptid eine Zell-Linie eines Säugers ist.

6. Solubilisierter Rezeptor für das Gastrin-freisetzende Peptid in aktiver Form.

7. Rezeptor nach Anspruch 6, der in folgenden Schritten solubilisiert wird:

(1) Herstellen von Membranen aus einem Ausgangsmaterial eines Rezeptors für das Gastrin-freisetzende Peptid;

(2) Waschen dieser Membranen und Suspendieren dieser Membranen in einer Pufferlösung;

(3) Solubilisieren des Rezeptors für das Gastrin-freisetzende Peptid durch Inkubieren dieser Membranen in einer Lösung, der ein Detergens und ein löslicher Cholesterylester zugegeben wurde; und

(4) Einstellen der Detergens-Konzentration des Rezeptors für das Gastrin-freisetzende Peptid.

8. Rezeptor nach Anspruch 7, wobei das Ausgangsmaterial des Rezeptors für das Gastrin-freisetzende Peptid ein eukaryotischer oder prokaryotischer Wirt ist, der den rekombinierten Gastrin-freisetzenden Peptid-Rezeptor exprimiert.

**9.** Rezeptor nach Anspruch 7, wobei das Ausgangsmaterial des Rezeptors für das Gastrin-freisetzende Peptid eine Zell-Linie eines Säugers ist.

**10.** Rezeptor nach Anspruch 7, wobei das Detergens 3-[(3-Cholamidopropyl)dimethylammonio]-1-propansulfonat ist und der lösliche Cholesterylester Cholesterylhemisuccinat ist.

**11.** Rezeptor nach Anspruch 6, der Gastrin-freisetzendes Peptid mit einer Affinität von mindestens $K_D=10$ nM bindet.

**12.** Rezeptor nach Anspruch 6, mit den folgenden Eigenschaften:

verläuft als breites Band in einer Trägerelektrophorese auf Natrium-Dodecylsulphat-Polyacrylamidgel (SDS-PAGE-Elektrophorese) mit einem scheinbaren Molgewicht von ca. 70 bis 100 Kilodalton;

bindet sich selektiv an Polypeptide des Bombesin-Typs;

hat einen $K_D$-Wert von ca. 10-100 pM;

enthält keine gekoppelten G-Proteine;

enthält N-gebundene Kohlenhydrate;

hat im entglykolisierten Zustand ein scheinbares Molgewicht von 36±5 Kilodalton in der Trägerelektrophorese auf Natrium-Dodecylsulphat-Polyacrylamidgel; und

hat eine Teil-Aminosäuresequenz in der Nähe der N-terminalen Aminosäure von:

```
-Leu-Asn-Leu-Asp-Val-Asp-Pro-Phe-Leu-Ser-.
```

**13.** Solubilisierter und gereinigter Rezeptor für Gastrin-freisetzendes Peptid in aktiver Form, der im wesentlichen frei von kontaminierenden Verunreinigungen ist.

**14.** Rezeptor nach Anspruch 13, der in folgenden Schritten solubilisiert und gereinigt wird:

(1) Herstellen von Membranen aus einem Ausgangsmaterial eines Rezeptors für das Gastrin-freisetzende Peptid;

(2) Waschen dieser Membranen und Suspendieren dieser Membranen in einer Pufferlösung;

(3) Solubilisieren des Rezeptors für das Gastrin-freisetzende Peptid durch Inkubieren dieser Membranen in einer Lösung, der ein Detergens und ein löslicher Cholesterylester zugegeben wurde, um einen löslichen Extrakt zu bilden;

(4) Fällen des Rezeptors für das Gastrin-freisetzende Peptid aus dem löslichen Extrakt aus Verfahrensschritt (3), Gewinnung des Niederschlags durch Zentrifugierung und Resuspendierung des Niederschlags in einer Pufferlösung, die das Detergens und den löslichen Cholesterylester enthält, um einen löslichen Extrakt zu bilden;

(5) Aufbringen des löslichen Extrakts aus Verfahrensschritt (4) auf eine Weizenkeim-Agglutinin-Affinitätssäule unter Bildung eines Eluats;

(6) weiteres Fraktionieren des Eluats der Weizenkeim-Agglutinin-Säule aus Verfahrensschritt (5) auf einer Affinitätssäule für das Gastrin-freisetzende Peptid unter Bildung eines Eluats; und

(7) Dialysieren des Eluats der Affinitätssäule für das Gastrin-freisetzende Peptid aus Verfahrensschritt (6) und Aufbringen dieses Eluats auf eine zweite Affinitätssäule für Gastrin-freisetzendes Peptid.

**15.** Rezeptor nach Anspruch 14, der durch den weiteren Schritt einer Gelfiltration im Anschluß an Verfahrensschritt (7) solubilisiert und gereinigt wird.

**16.** Rezeptor nach Anspruch 13, mit den folgenden Eigenschaften:

verläuft als breites Band in der Trägerelektrophorese auf Natrium-Dodecylsulphat-Polyacrylamidgel mit einem scheinbaren Molgewicht von ca. 70 bis 100 Kilodalton;

bindet sich selektiv an Polypeptide des Bombesin-Typs;

hat einen $K_D$-Wet von ca. 10-100 pM;

enthält keine gekoppelten G-Proteine;

enthält N-gebundene Kohlenhydrate;

hat im entglykolisierten Zustand ein scheinbares Molgewicht von 36±5 Kilodalton in der Trägerelektrophorese auf Natrium-Dodecylsulphat-Polyacrylamidgel; und

hat eine Teil-Aminosäuresequenz in der Nähe der N-terminalen Aminosäure von:

```
-Leu-Asn-Leu-Asp-Val-Asp-Pro-Phe-Leu-Ser-.
```

**17.** Antikörper mit einer Bindungsaffinität an einen in der Natur vorkommenden Rezeptor für das Gastrin-freisetzende Peptid.

**18.** Antikörper nach Anspruch 17, die auf den GRP-Rezeptor oder Fragmente davon ansprechen.

**19.** Antikörper nach Anspruch 17, die mit Radionukliden konjugieren.

**20.** Screeningverfahren für Verbindungen, die eine Bindungsaffinität an den Rezeptor für das Gastrin-freisetzende Peptid haben, wobei das Verfahren die Verwendung eines Ausgangsmaterials eines Rezeptors für das Gastrin-freisetzende Peptid, die löslich und aktiv und im wesentlichen frei von anderem Bindungsprotein für Gastrin-freisetzendes Peptid ist, in einem Bindungsassay Rezeptor/markierte Verbindung umfaßt, mit folgenden Verfahrensschritten:

(1) in Kontakt bringen und Inkubieren des Ausgangsmaterials des Rezeptors für das Gastrin-freisetzende Peptid mit einer markierten Verbindung, die eine bekannte Bindungsaffinität an den Rezeptor für das Gastrin-freisetzende Peptid hat, und einer Testverbindung, deren Bindungsaffinität an den Rezeptor für das Gastrin-freisetzende Peptid ausgewertet werden soll;

(2) Trennen gebundener von freier markierter Verbindung; und

(3) Messen des Bindungsgrades der markierten Verbindung, wobei dieser Wert umgekehrt proportional zum Betrag der Bindung der Testverbindung ist.

**21.** Verfahren nach Anspruch 20, wobei das Ausgangsmaterial des Rezeptors für das Gastrin-freisetzende Peptid eine eukaryotische oder prokaryotische Wirtszelle ist, die mit Molekülen rekombinierter DNA unverändert transformiert wurde, die den Rezeptor für das Gastrin-freisetzende Peptid exprimieren.

**22.** Verfahren nach Anspruch 20, wobei das Ausgangsmaterial des Rezeptors für das Gastrin-freisetzende Peptid Membranen aus einer eukaryotischen oder prokaryotischen Zelle sind, die mit DNA-Vektoren unverändert transformiert wurde, welche die Expression des Rezeptors für das Gastrin-freisetzende Peptid steuern.

**23.** Verfahren nach Anspruch 20, wobei der solubilisierte Rezeptor gereinigt wird.

**24.** Verfahren nach Anspruch 20, wobei die markierte Verbindung ein Ligand oder ein Antikörper ist.

**25.** Kit zur Bestimmung der Bindungsaffinität einer Testverbindung an den Rezeptor für das Gastrin-freisetzende Peptid, umfassend eine markierte Verbindung, deren Bindungsaffinität an den Rezeptor für das Gastrin-freisetzende Peptid bekannt ist, den Rezeptor für das Gastrin-freisetzende Peptid in aktiver Form, der im wesentlichen frei von anderem Bindungsprotein für das Gastrin-freisetzende Peptid ist, und ein Mittel zum Trennen gebundener von freier markierter Verbindung.

**26.** Kit nach Anspruch 25, wobei das Mittel zum Trennen eine feste Phase zur Immobilisierung des Rezeptors für das Gastrin-freisetzende Peptid ist.

**27.** Kit nach Anspruch 25, wobei die markierte Verbindung ein Ligand oder ein Antikörper ist.

**28.** Verfahren zur Bestimmung der Konzentration eines Rezeptors für das Gastrin-freisetzende Peptid in einer Probe, folgende Schritte umfassend:

(1) Herstellen von Membranen aus einer Probe, die aus einem Ausgangsmaterial eines Rezeptors für das Gastrin-freisetzende Peptid besteht;

(2) Waschen dieser Membranen und Suspendieren dieser Membranen in einer Pufferlösung;

(3) Solubilisieren des Rezeptors für das Gastrin-freisetzende Peptid durch Inkubieren der Membranen in einer Lösung, der ein Detergens und ein löslicher Cholesterylester zugegeben wurde;

(4) Einstellen der Detergens-Konzentration des solubilisierten Rezeptors für das Gastrin-freisetzende Peptid;

(5) in Kontakt bringen und Inkubieren der verdünnten Lösung mit radioaktiv markiertem Gastrin-freisetzenden Peptid zur Bildung von Komplexen aus Gastrinfreisetzendem Peptid: Gastrin-freisetzender Peptid-Rezeptor;

(6) Gewinnen dieser Komplexe; und

(7) Messen der Radioaktivität der gewonnen Komplexe.

**29.** Kit zur Bestimmung der Konzentration eines Rezeptors für das Gastrin-freisetzende Peptid in einer Probe, umfassend eine markierte Verbindung mit einer bekannten Bindungsaffinität an den Rezeptor für das Gastrin-freisetzende Peptid, den Rezeptor für das Gastrin-freisetzende Peptid in aktiver Form und im wesentlichen frei von anderem Bindungsprotein für das Gastrin-freisetzende Peptid sowie ein Mittel zur Trennung gebundener von freier markierter Verbindung.

**30.** Kit nach Anspruch 29, wobei das Mittel zum Trennen eine feste Phase zur Immobilisierung des Rezeptors für das Gastrin-freisetzende Peptid ist.

**31.** Kit nach Anspruch 29, wobei die markierte Verbindung ein Ligand oder ein Antikörper ist.

**32.** Antikörper mit Bindungsaffinität an in der Natur vorkommenden Rezeptor für das Gastrin-freisetzende Peptid zur Verwendung bei der Behandlung einer Krankheit oder einer Störung, die mit einer abnormalen Expression oder abnormalem Triggern des Rezeptors für das Gastrin-freisetzende Peptid einhergeht.

**33.** Antikörper nach Anspruch 32, wobei der Rezeptor für das Gastrin-freisetzende Peptid in aktiver Form solubilisiert ist und die Antikörper Epitope erkennen, die nur im aktiven Rezeptor für das Gastrin-freisetzende Peptid vorhanden sind.

**Revendications**

**1.** Procédé pour solubiliser le récepteur du peptide de libération de la gastrine, sous forme active, qui comprend les étapes consistant à :

(1) préparer des membranes à partir d'une source du récepteur du peptide de libération de la gastrine ;

(2) laver lesdites membranes et mettre en suspension lesdites membranes dans un tampon ;

(3) solubiliser le récepteur du peptide de libération de la gastrine en incubant lesdites membranes dans une solution à laquelle on a ajouté un détergent et un ester cholestérylique soluble, et

(4) ajuster la concentration du détergent dans ledit récepteur solubilisé du peptide de libération de la gastrine.

2. Procédé selon la revendication 1, dans lequel ladite source du récepteur du peptide de libération de la gastrine est un hôte eucaryote ou procaryote exprimant le récepteur recombinant du peptide de libération de la gastrine.

3. Procédé pour solubiliser et purifier le récepteur du peptide de libération de la gastrine, sous forme active, qui comprend les étapes consistant à :

(1) préparer des membranes à partir d'une source du récepteur du peptide de libération de la gastrine ;

(2) laver lesdites membranes et mettre en suspension lesdites membranes dans un tampon ;

(3) solubiliser le récepteur du peptide de libération de la gastrine en incubant lesdites membranes dans une solution à laquelle on a ajouté un détergent et un ester cholestérylique soluble, pour former un extrait soluble ;

(4) provoquer la précipitation, à partir de l'extrait soluble de l'étape (3), du récepteur du peptide de libération de la gastrine, recueillir le précipité par centrifugation et remettre en suspension ledit précipité dans un tampon contenant ledit détergent et l'ester cholestérylique soluble, pour former un extrait soluble ;

(5) appliquer l'extrait soluble de l'étape (4) sur une colonne d'affinité à l'agglutinine du germe de blé, pour former un éluat ;

(6) soumettre à un fractionnement supplémentaire l'éluat de la colonne à l'agglutinine du germe de blé obtenu à l'étape (5), sur une colonne d'affinité au peptide de libération de la gastrine, pour former un éluat ; et

(7) dialyser l'éluat de la colonne d'affinité au peptide de libération de la gastrine, obtenu à l'étape (6), et appliquer ledit éluat à une deuxième colonne d'affinité au peptide de libération de la gastrine.

4. Procédé selon la revendication 3, qui comprend en outre une étape de filtration sur gel après l'étape (7).

5. Procédé selon la revendication 3, dans lequel ladite source du récepteur du peptide de libération de la gastrine est une lignée cellulaire de mammifère.

6. Récepteur solubilisé du peptide de libération de la gastrine, sous une forme active.

7. Récepteur selon la revendication 6, que l'on solubilise :

(1) en préparant des membranes à partir d'une source du récepteur du peptide de libération de la gastrine ;

(2) en lavant lesdites membranes et en mettant en suspension lesdites membranes dans un tampon ;

(3) en solubilisant le récepteur du peptide de libération de la gastrine en incubant lesdites membranes dans une solution à laquelle on a ajouté un détergent et un ester cholestérylique soluble ; et

(4) en ajustant la concentration du détergent dans ledit récepteur du peptide de libération de la gastrine.

8. Récepteur selon la revendication 7, dans lequel ladite source du récepteur du peptide de libération de la gastrine est un hôte eucaryote ou procaryote exprimant le récepteur recombinant du peptide de libération de la gastrine.

9. Récepteur selon la revendication 7, dans lequel ladite source du récepteur du peptide de libération de la gastrine est une lignée cellulaire de mammifère.

10. Récepteur selon la revendication 7, dans lequel ledit détergent est un 3-[(3-cholamidopropyl)diméthylammonio]-1-propanesulfonate et ledit ester cholestérylique soluble est l'hémisuccinate de cholestéryle.

11. Récepteur selon la revendication 6, qui fixe le peptide de libération de la gastrine avec une affinité d'au moins $K_D$ = 10 nM.

12. Récepteur selon la revendication 6, ayant les caractéristiques suivantes :

il passe sous forme d'une bande large sur SDS-PAGE avec une masse moléculaire apparente d'environ 70 à 100 kilodaltons ;

il se fixe sélectivement aux polypeptides du type bombésine ;

il a un $K_D$ d'environ 10 à 100 pM ;

il est exempt de protéines G couplées ;

il contient des hydrates de carbone liés à l'azote ;

après déglycosylation, il a une masse moléculaire apparente de 36±5 kilodaltons sur SDS-PAGE ; et

il présente au voisinage du site N-terminal la séquence partielle d'acides aminés suivante :

**–Leu–Asn–Leu–Asp–Val–Asp–Pro–Phe–Leu–Ser–.**

13. Récepteur solubilisé et purifié du peptide de libération de la gastrine, sous une forme active, qui est essentiellement exempt d'impuretés contaminantes.

14. Récepteur selon la revendication 13, que l'on solubilise et purifie par un procédé consistant à :

(1) préparer des membranes à partir d'une source du récepteur du peptide de libération de la gastrine ;

(2) laver lesdites membranes et mettre en suspension lesdites membranes dans un tampon ;

(3) solubiliser le récepteur du peptide de libération de la gastrine en incubant lesdites membranes dans une solution à laquelle on a ajouté un détergent et un ester cholestérylique soluble, pour former un extrait soluble ;

(4) provoquer la précipitation, à partir de l'extrait soluble de l'étape (3), du récepteur du peptide de libération de la gastrine, recueillir le précipité par centrifugation et remettre en suspension ledit précipité dans un tampon contenant ledit détergent et l'ester cholestérylique soluble, pour former un extrait soluble ;

(5) appliquer l'extrait soluble de l'étape (4) sur une colonne d'affinité à l'agglutinine du germe de blé, pour former un éluat ;

(6) soumettre à un fractionnement supplémentaire l'éluat de la colonne à l'agglutinine du germe de blé obtenu à l'étape (5), sur une colonne d'affinité au peptide de libération de la gastrine, pour former un éluat ; et

(7) dialyser l'éluat de la colonne d'affinité au peptide de libération de la gastrine, obtenu à l'étape (6), et appliquer ledit éluat à une deuxième colonne d'affinité au peptide de libération de la gastrine.

15. Récepteur selon la revendication 14, qui est solubilisé et purifié par l'étape supplémentaire de filtration sur gel après l'étape (7).

16. Récepteur selon la revendication 13, ayant les caractéristiques suivantes :

il passe sous forme d'une bande large sur SDS-PAGE avec une masse moléculaire apparente d'environ 70 à 100 kilodaltons ;

il se fixe sélectivement aux polypeptides du type bombésine ;

il a un $K_D$ d'environ 10 à 100 pM ;

il est exempt de protéines G couplées ;

il contient des hydrates de carbone liés à l'azote ;

après déglycosylation, il a une masse moléculaire apparente de 36±5 kilodaltons en SDS-PAGE ; et

il présente au voisinage du site N-terminal la séquence partielle d'acides aminés suivante :

**–Leu–Asn–Leu–Asp–Val–Asp–Pro–Phe–Leu–Ser–.**

17. Anticorps ayant une affinité de fixation vis-à-vis d'un récepteur naturel du peptide de libération de la gastrine.

18. Anticorps selon la revendication 17, qui sont dirigés contre le récepteur du GRP, ou ses fragments.

19. Anticorps selon la revendication 17, qui sont conjugués à des radionucléides.

20. Procédé de sélection de composés ayant une affinité de fixation vis-à-vis du récepteur du peptide de libération de la gastrine, qui consiste à utiliser une source du récepteur du peptide de libération de la gastrine, qui est soluble et actif et essentiellement exempt d'une autre protéine de fixation du peptide de libération de la gastrine, dans une analyse de fixation récepteur/composé marqué, qui comprend les étapes consistant à :

(1) mettre en contact et incuber ladite source du récepteur du peptide de libération de la gastrine avec un composé marqué ayant une affinité connue de fixation au récepteur du peptide de libération de la gastrine, et un composé d'essai dont il s'agit d'évaluer l'affinité de fixation au récepteur du peptide de libération de la gastrine ;

(2) séparer le composé marqué lié du composé marqué libre ; et

(3) mesurer le degré de fixation du composé marqué, cette valeur étant inversement proportionnelle à la quantité de fixation du composé d'essai.

21. Procédé selon la revendication 20, dans lequel ladite source du récepteur du peptide de libération de la gastrine est une cellule hôte eucaryote ou procaryote, qui a été transformée d'une manière stable avec des molécules d'ADN recombinant qui expriment le récepteur du peptide de libération de la gastrine.

22. Procédé selon la revendication 20, dans lequel ladite source du récepteur du peptide de libération de la gastrine est constituée de membranes provenant d'une cellule eucaryote ou procaryote qui a été transformée d'une manière stable avec des vecteurs à ADN dirigeant l'expression du récepteur du peptide de libération de la gastrine.

23. Procédé selon la revendication 20, dans lequel ledit récepteur solubilisé est purifié.

24. Procédé selon la revendication 20, dans lequel ledit composé marqué est un ligand ou un anticorps.

25. Trousse pour déterminer l'affinité de fixation d'un composé d'essai au récepteur du peptide de libération de la gastrine, comprenant un composé marqué ayant une affinité de fixation connue au récepteur du peptide de libération de la gastrine, le récepteur du peptide de libération de la gastrine sous une forme active, et essentiellement exempt d'une autre protéine de fixation du peptide de libération de la gastrine, et un moyen pour séparer le composé marqué lié du composé marqué libre.

26. Trousse selon la revendication 25, dans laquelle lesdits moyens de séparation sont constitués par une phase solide permettant d'immobiliser le récepteur du peptide de libération de la gastrine.

27. Trousse selon la revendication 25, dans laquelle ledit composé marqué est un ligand ou un anticorps.

28. Procédé pour déterminer la concentration du récepteur du peptide de libération de la gastrine dans un échantillon, qui comprend les étapes consistant à :

(1) préparer des membranes à partir d'un échantillon constitué d'une source du récepteur du peptide de libération de la gastrine ;
(2) laver lesdites membranes et mettre en suspension lesdites membranes dans un tampon ;
(3) solubiliser le récepteur du peptide de libération de la gastrine en incubant lesdites membranes dans une solution à laquelle on a ajouté un détergent et un ester cholestérylique soluble ;
(4) ajuster la concentration du détergent dans ledit récepteur solubilisé du peptide de libération de la gastrine ;
(5) mettre en contact et incuber ladite dilution avec un peptide radiomarqué de libération de la gastrine, pour former des complexes peptide de libération de la gastrine/récepteur du peptide de libération de la gastrine ;
(6) récupérer lesdits complexes ; et
(7) mesurer la radioactivité desdits complexes récupérés.

29. Trousse pour déterminer la concentration du récepteur du peptide de libération de la gastrine dans un échantillon, comprenant un composé marqué ayant une affinité connue de fixation pour le récepteur du peptide de libération de la gastrine, le récepteur du peptide de libération de la gastrine sous une forme active et essentiellement exempt d'une autre protéine de fixation du peptide de libération de la gastrine, et des moyens pour séparer le composé marqué lié du composé marqué libre.

30. Trousse selon la revendication 29, dans laquelle lesdits moyens de séparation sont constitués par une phase solide destinée à immobiliser le récepteur du peptide de libération de la gastrine.

31. Trousse selon la revendication 29, dans laquelle ledit composé marqué est un ligand ou un anticorps.

32. Anticorps ayant une affinité de fixation vis-à-vis du récepteur naturel du peptide de libération de la gastrine, pour utilisation dans le traitement d'une maladie ou d'un trouble associé à l'expression anormale ou au déclenchement anormal du récepteur du peptide de libération de la gastrine.

33. Anticorps selon la revendication 32, dans lesquels ledit récepteur du peptide de libération de la gastrine est solubilisé sous une forme active, et lesdits anticorps reconnaissent des épitopes qui ne sont présents que dans le récepteur actif du peptide de libération de la gastrine.

FIG.1

FIG.2

FIG.3

FIG.4

**FIG.6**

MW

200 —

116 —
97 —

68 —

43 —

A B C D E

MW (kDa)

200 —

100 —

69 —

46 —

**FIG.5**

STRIP 109

7.2 kb →

3.1 kb →

—28 S

—18 S

**FIG.10**

HFL  BALB SWISS

← 7.2 kb
—28 S
← 3.0 kb
—18 S

**FIG.12**

EP 0 524 173 B1

**FIG.7**

**FIG.9**

-377 AAAACTGCAGCCAGAGAGACTCAGTCTAGGATGGAGGTAGGAAGAGC

-277 TTGTTAACTTAGTGAATGTACAGATGTATTGCTTGCTGGTGGTGTGA

-177 GTGTGTGTGTGTGTGTGTGTGTGTGTGTGTGTGTGTGAATTCAGAGT

-77 CCTTCAGCGCCTAACTGAAAAACCCAGAAGTTACAAAGCAGCATCTT

23 CCTGAACTTGGACGTGGACCCTTTCCTGTCCTGCAACGACACCTTCA
   sLeuAsnLeuAspValAspProPheLeuSerCysAsnAspThrPheA

123 TATGTCATCCCTGCAGTTTATGGGCTTATCATCGTGATAGGTCTTAT
   TyrValIleProAlaValTyrGlyLeuIleIleValIleGlyLeuIl

223 TGCCAAACCTGTTCATCTCTAGCCTGGCTTTGGGAGACCTGCTGCTG
   alProAsnLeuPheIleSerSerLeuAlaLeuGlyAspLeuLeuLeu

323 ATTTGGCAGAATTGGCTGCAAACTGATCCCCTTTATACAACTTACTT
   uPheGlyArgIleGlyCysLysLeuIleProPheIleGlnLeuThrS

423 GCCATTGTACGGCCAATGGATATCCAGGCATCCCATGCCCTGATGAA
   AlaIleValArgProMetAspIleGlnAlaSerHisAlaLeuMetLy

# FIG.8A

37

```
TGAGACAAAGTGGGCTTAATTCTAAGCTTTTCTTCAGGCTGAGTTTCTGTTGC  -278

     .              •              •              •              •              •
AGGCTGGGACAGAACCAACATCAACAAACTGAGCTAGAGTTTGGAATACCAGT  -178

     •              •              •              •              •              •
GTTTTAAAGAGAGATCAAGAGGCTCACACAGATCAGCGAGCCTAACTGACAAA   -78

     •              •              •              •              •              •
GAAGGCGCATTTGAAGAGAGAAGCTTTGAGATGGCTCCAAATAATTGTTCCCA    22
                                   · MetAlaProAsnAsnCysSerHi

     •              •              •              •              •              •
ATCAAAGTCTGAGTCCCCCCAAGATGGACAACTGGTTTCACCCGGGCTTCATC   122
snGlnSerLeuSerProProLysMetAspAsnTrpPheHisProGlyPheIle

     •              •              •              •              •              •
TGGCAACATCACGCTCATCAAGATCTTCTGCACGGTCAAGTCCATGCGAAACG   222
eGlyAsnIleThrLeuIleLysIlePheCysThrValLysSerMetArgAsnV

     •              •              •              •              •              •
CTGGTGACATGCGCCCCTGTGGATGCCAGCAAGTACCTGGCTGACAGGTGGCT   322
LeuValThrCysAlaProValAspAlaSerLysTyrLeuAlaAspArgTrpLe

     •              •       III      •              •              •              •
CAGTGGGGGGTGTCTGTCTTCACACTTACGGCACTGTCAGCTGACAGGTACAAA   422
erValGlyValSerValPheThrLeuThrAlaLeuSerAlaAspArgTyrLys

     •              •              •       IV       •              •              •
GATCTGTCTCAAAGCTGCTTTGATCTGGATTGTCTCTATGTTGTTGGCCATCC   522
sIleCysLeuLysAlaAlaLeuIleTrpIleValSerMetLeuLeuAlaIleP
```

# FIG.8AA

523 CAGAGGCTGTGTTTTCTGACCTCCACCCCTTCCATGTGAAAGATACC
roGluAlaValPheSerAspLeuHisProPheHisValLysAspThr

623 TAAAATCCATTCCATGGCTTCCTTTCTGGTTTTCTACGTTATCCCAC
oLysIleHisSerMetAlaSerPheLeuValPheTyrValIleProL

723 GCCTACAATCTTCCCGTGGAAGGCAATATACATGTCAAGAAGCAGAT
AlaTyrAsnLeuProValGluGlyAsnIleHisValLysLysGlnIl

823 CCTTCTGCTGGCTCCCCAACCATGTCATCTACCTGTACCGTTCCTAC
laPheCysTrpLeuProAsnHisValIleTyrLeuTyrArgSerTyr

923 CCACCTCCTGGCCTTCACCAACTCCTGTGTGAACCCCTTTGCTCTTT
aHisLeuLeuAlaPheThrAsnSerCysValAsnProPheAlaLeuT

1023 CAGCCTGGCCTGATGAACAGGTCCCACAGCACAGGCAGAAGTACCAC
GlnProGlyLeuMetAsnArgSerHisSerThrGlyArgSerThrTh

1123 ACAGAAATATCTGTCATGAGGGGTATGTCTAGACTAAACTTCAACCT
snArgAsnIleCysHisGluGlyTyrValEnd

1223 TTGATTGTTGTCTCTATATCTTCTGAAGACTCTTCAGGGGGATGAGT

# FIG.8B

```
AACCAAACCTTCATTAGTTGTGCCCCCTACCCACACTCCAATGAGCTACACCC    622
AsnGlnThr PheIleSerCysAlaProTyrProHisSerAsnGluLeuHisPr


  •        •         •         •         •         •

TGGCGATCATCTCTGTCTACTACTACTTCATTGCCCGAAATCTGATTCAGAGT    722
euAlaIleIleSerValTyrTyrTyrPheIleAlaArgAsnLeuIleGlnSer


  •        •         •         •         •  VI      •

CGAATCCCGGAAGCGGCTTGCCAAGACAGTACTGGTGTTTGTGGGCCTCTTTG    822
eGluSerArgLysArgLeuAlaLysThrValLeuValPheValGlyLeuPheA


  •        •         •         •         •  VII     •

CACTACTCTGAAGTGGACACCTCCATGCTCCACTTTGTCACCAGCATCTGTGC    922
HisTyrSerGluValAspThrSerMetLeuHisPheValThrSerIleCysAl


  •        •         •         •         •         •

ATCTGCTGAGCAAGAGCTTCAGGAAGCAGTTCAACACTCAACTTCTCTGCTGC   1022
yrLeuLeuSerLysSerPheArgLysGlnPheAsnThrGlnLeuLeuCysCys


  •        •         •         •         •        • •

CTGCATGACCTCCTTCAAGAGCACTAACCCCTCGGCTACCTTTAGCCTCATCA   1122
rCysMetThrSerPheLysSerThr AsnProSer AlaThrPheSerLeuIleA


  •        •         •         •         •         •

TGCCTCTAAAGGAACTCCTGGTATTGTTCTACAGATGTCCAGGGGCCCTGAGA   1222


  •        •         •         •         •         •

GATACAGACGGATGGGAAAGATGTCCAAATGCACCAATCACCATTGTATCTCA   1322
```

# FIG.8BB

EP 0 524 173 B1

```
  1 MAPNNCSHLNLDVDPFLSCNDTFNQSLSPPKMDNWFHPGFIYVIPAVYGL  50 ●
          |  | |       | |                        | |
  1 .MGACVVMTDINISSGLDSNATGITAFSMPGWQ.......LALWTAAYLA  42 ▲

 51 IIVIGLIGNITLIKIFCTVKSMRNVPNLFISSLALGDLLLLVTCAPVDAS 100
       || | | |      || | | || ||| ||        |
 43 LVLVAVMGNATVIWIILAHQRMRTVTNYFIVNLALADLCMAAFNAAFNFV  92

101 KYLADRWLFGRIGCKLIPFIQLTSVGVSVFTLTALSADRYKAIVRPMDIQ 150
       | |||   |        |    ||    || |||| ||| |
 93 YASHNIWYFGRAFCYFQNLFPITAMFVSIYSMTAIAADRYMAIVHPFQPR 142

151 ASHALMKICLKAALIWIVSMLLAIPEAVFSDLHPFHVKDTNQTFISCAPY 200
       |         | || |   ||| |    |       |   |    |
143 LSAPGTRAVI..AGIWLVALALAFPQCFYSTI....TTDEGATKCVVAWP 186

201 PHSNELHPKIHSMASFLVFYVIPLAIISVYYYFIARNLIQSAYNLPVEGN 250
       |                 |  ||    | | | |    |
187 EDSGGKMLLLYHLIVIALIYFLPLVVMFVAYSVIGLTLWRRSVPGHQAHG 236

251 IHVKKQIESRKRLAKTVLVFVGLFAFCWLPNHVIYLYRSYHYSEVDTSML 300
         |     ||     |   || |||| |             |
237 ANL.RHLQAKKKFVKTMVLVVVTFAICWLPYHLYFILGTF...QEDIYCH 282

301 HFVTSICAHLLAFTNSCV..NPFALYLLSKSFRKQFNTQLLCCQ......  342
       |         |      ||     |     || |     ||
283 KFIQQVYLALFWLAMSSTMYNPIIYCCLNHRFRSGFRLAFRCCPWVTPTE 332

343 .........PGLMNRSHSTGRSTTCMTSFKSTNPSATFSLINRNICHEGY 383
            | |     |       |     |      |
333 EDKMELTYTPSL...STRVNRCHTKEIFFMSGDVAPSEAVNGQAESPQAG 379

384 V*.... 385
     |
380 VSTEP* 385
```

LEGEND:
● GRP RECEPTOR
▲ SUBSTANCE K RECEPTOR

**FIG.11**

41

**FIG.13**

$10^{-9}$ M GRP